# EUROPEAN PATENT SPECIFICATION

(11) **EP 0 434 750 B1**
(45) Date of publication and mention of the grant of the patent: **09.07.1997**
(21) Application number: 89910980.5
(22) Date of filing: 12.09.1989
(51) Int. Cl.: A61K 31/40

(54) **Medicament for treating degenerative diseases of the nervous system**
Zusammensetzung zur Behandlung von degenerativen Krankheiten des Nervensystems
Medicament pour le traitement de maladies degéneratives du system nerveux

(30) Priority: 13.09.1988 US 243736; 31.03.1989 US 331123
(43) Date of publication of application: 03.07.1991
(73) Proprietor: Biosource Technologies, Inc., Vacaville, CA 95688 (US)
(72) Inventor: BERLINER, David, L., Atherton, CA 94025 (US); ERWIN, Robert, L., San Francisco, CA 94107 (US); MCGEE, David, R., Vacaville, VA 95687 (US)
(74) Representative: Schüssler, Andrea, Dr.
(86) International application number: US8903859
(87) International publication number: WO9002551

(56) References cited:
- MEDLINE AN 06029385, "Current Concepts in Clinical Therapeutics, Parkinsons Disease"
- MEDLINE AN 06479503, "Studies on the Melanin Affinity of Selegilin (Deprenyl) and other Amphetamine Derivatives"

## Description

The present invention relates to the use of an active substance in the manufacture of a medicament to treat degenerative diseases of the nervous system wherein the active substance causes an increase in the concentration of melanin in the effected nervous system tissue and the active substance is selected from melanin, melanin derivatives in combination with melanin, tyrosinase, tyrosinase gene or melanin-concentrating hormone, the enzyme tyrosinase, which catalyzes the reaction wherein naturally occurring melanin precursors are converted to melanin, tyrosinase gene, melanin-concentrating hormone and combinations thereof. Examples of such diseases include Parkinson's disease, Alzheimer's disease, retinitis pigmentosa and dementia.

### A. Nervous System

The nervous system and epidermis have a common embryological basis and several common features.

### 1. Embryological Basis

During gastrulation the single layer of cells comprising the blastoderm migrate and fold to form the three germinal layers -- ectoderm, endoderm and mesoderm. The germinal layers are the rudiments from which organs of the plant or animal develop. The ectoderm, for example, gives rise to the epidermis, central nervous system, i.e., the brain, spinal cord, spinal ganglia and nerves, various sensory organs and neural crest cell derivatives that includes cerebro-spinal ganglia and melanocytes.

Although by definition the ectoderm is the outermost of the germinal layers, it is not long during gastrulation that by migration and invagination cells once on the surface are displaced into the interior of the developing embryo. Because nervous tissue and epidermis have a shared origin, it is not uncommon for embryologic diseases to affect seemingly unrelated organs such as brain and skin.

Another example of cell migration and invagination during development is the adrenal gland. The medulla of the gland is a highly specialized adjunct to the sympathetic nervous system and derived from the ectoderm. The cortex, on the other hand, is derived from endoderm and mesoderm. The adrenal medulla secretes the catecholamines adrenaline (epinephrine) and noradrenaline (norepinephrine).

### 2. Cell Structure and Coloration

Early in development, the neural crest cells lie dorsal to the neural tube. Soon they migrate laterally and ventrally, basically associating with ectodermally-derived structures as in the areas of the epidermis-dermis junction.

Melanocytes are the cells found in the skin and are epidermal derivatives that are responsible for coloration. Those cells have polygonal cell bodies and long dendritic processes that ramify between epithelial cells throughout the lower strata of the epidermis. Pigmented cells are not restricted to cutaneous structures but can be found associated with various internal structures of ectodermal origin, as in the brain, spinal cord or adrenal medulla.

### 3. Nervous System

Neurons have a polygonal cell body and two types of arborizing processes, the axon and one or more dendrites. One region of the brain is called the substantia nigra (for black substance) because of its highly pigmented character. Many of the neurons of the substantia nigra contain significant quantities of melanin, and it is the melanin that confers on those cells the dark coloration. It has been seen that cell death in the normal substantia nigra appears to be related to the content of neuromelanin per cell. Mann, D.M. et al., Brain 97, 489 (1974).

The substantia nigra is one region of the brain that is involved in the coordination (planning and programming) of neural signals for gross and slow, steady movements (ramp movements) and posture. The substantia nigra is part of that portion of the brain known as the basal ganglia which is itself part of the midbrain.

Two other highly pigmented areas of the brain are the locus ceruleus and the pituitary gland. The locus ceruleus is an eminence in the superior angle of the floor of the fourth ventricle. The hypophysis (pituitary gland), like the adrenal gland, arises from two embryological sources. The anterior pituitary arises as an epithelial outgrowth from the roof of the mouth. One of the hormones that it secretes is melanocyte stimulating hormone. The posterior pituitary is derived from a downgrowth of hypothalamic nerve tracts.

### B. Degenerative Diseases of the Nervous System

The term "degenerative" as applied to diseases of the nervous system is used to designate a group of disorders in which there is gradual, generally symmetric, relentlessly progressive wasting away of neurons, for reasons still unknown. Many of the conditions so designated depend on genetic factors and thus appear in more than one member of the same family. This general group of diseases is therefore frequently referred to as heredodegenerative. A number of other conditions, not apparently differing in any fundamental way from the hereditary disorders, occur only sporadically, i.e., as isolated instances in a given family. For all diseases of this class William Gowers in 1902 suggested the now-familiar term "abiotrophy," by which he meant "defective vital endurance" of the structures affected, leading to their premature death. This term, of course, tells nothing of the true nature of the defects. It is to be assumed that their basis must be some disorder of the metabolism of the parts involved.

Within relatively recent times there has been some elucidation of the nature of a number of metabolic nervous disorders which, in their symmetric distribution and gradually progressive course, resemble the degenerative diseases under discussion. It is to be expected that with advances in knowledge others of the latter group will eventually find their place in the metabolic category. The degenerative diseases of the nervous system manifest themselves by a number of common syndromes easily distinguished by their clinical attributes, the recognition of which can assist the clinician in arriving at the diagnosis of a disorder of this class.

### 1. General Considerations

It is a characteristic of the degenerative diseases that they begin insidiously and run a gradually progressive course which may extend over many years. The earliest changes may be so slight that it is frequently impossible to assign any precise time of onset. However, as with other gradually developing conditions, the patient or his family may give a history implying an abrupt appearance of disability. This is particularly likely to occur if there has been an injury, or if some other dramatic event has taken place in the patient's life, to which illness might conceivably be related. In such a case, skillful taking of the history may bring out that the patient or family has suddenly become aware of a condition which had, in fact, already been present for some time but had passed unnoticed. Whether trauma or other stress may bring on or aggravate one of the degenerative diseases is still a question that cannot be answered with certainty. From all that is known it would seem highly improbable that this could happen. In any event, it must be kept in mind that the disease processes under discussion by their very nature develop spontaneously without relation to external factors.

Family history of degenerative nervous diseases is a significant feature of this class of diseases. Another significant feature is that in general their ceaselessly progressive course is uninfluenced by all medical or surgical measures. Dealing with a patient with this type of illness is often, therefore, an anguishing experience for all concerned. Yet symptoms can often be alleviated by wise and skillful management, and the physician's kindly interest may be of great help even when curative measures cannot be offered.

The bilaterally symmetric distribution of the changes brought about by these diseases has already been mentioned. This feature alone may serve to distinguish conditions in this group from many other diseases of the nervous system. At the same time, it should be pointed out that, in the earliest stages, greater involvement on one side or in one limb is not uncommon. Sooner or later, however, despite the asymmetric beginning, the inherently generalized nature of the process asserts itself.

A striking feature of a number of disorders of this class is the almost selective involvement of anatomically or physiologically related systems of neurons. This is clearly exemplified in amyotrophic lateral sclerosis, in which the process is almost entirely limited to cortical and spinal motor neurons, and in certain types of progressive ataxia, in which the Purkinje cells of the cerebellum are alone affected. Many other examples could be cited (e.g., Friedreich's ataxia) in which certain neuronal systems disintegrate, leaving others perfectly intact. An important group of the degenerative diseases has therefore been called "system diseases" ("progressive cerebrospinal system atrophies"), and many of these are strongly hereditary. It must be realized, however, that selective involvement of neuronal systems is not exclusively a property of the degenerative group, since several disease processes of known cause have similarly circumscribed effects on the nervous system. Diphtheria toxin, for instance, selectively attacks the myelin of the peripheral nerves, and triorthocresyl phosphate affects particularly the corticospinal tracts in the spinal cord as well as the peripheral nerves. Another example is the special vulnerability of the Purkinje cells of the cerebellum to hyperthermia. On the other hand, several of the conditions included among the degenerative diseases are characterized by pathologic changes that are diffuse and unselective. These exceptions nevertheless do not detract from the importance of affection of particular neuronal systems as a distinguishing feature of many of the diseases under discussion.

Since etiologic classification is impossible, sub-division of the degenerative diseases into individual syndromes rests on descriptive criteria, based largely on pathologic anatomy but to some extent on clinical aspects as well. In the terms used to designate many of these syndromes, the names of a number of distinguished neurologists and neuropathologists are commemorated. A useful way of keeping in mind the various disease states is to group them according to the outstanding clinical features that may be found in an actual case. The classification outlined in Table 1 is based on such a plan.

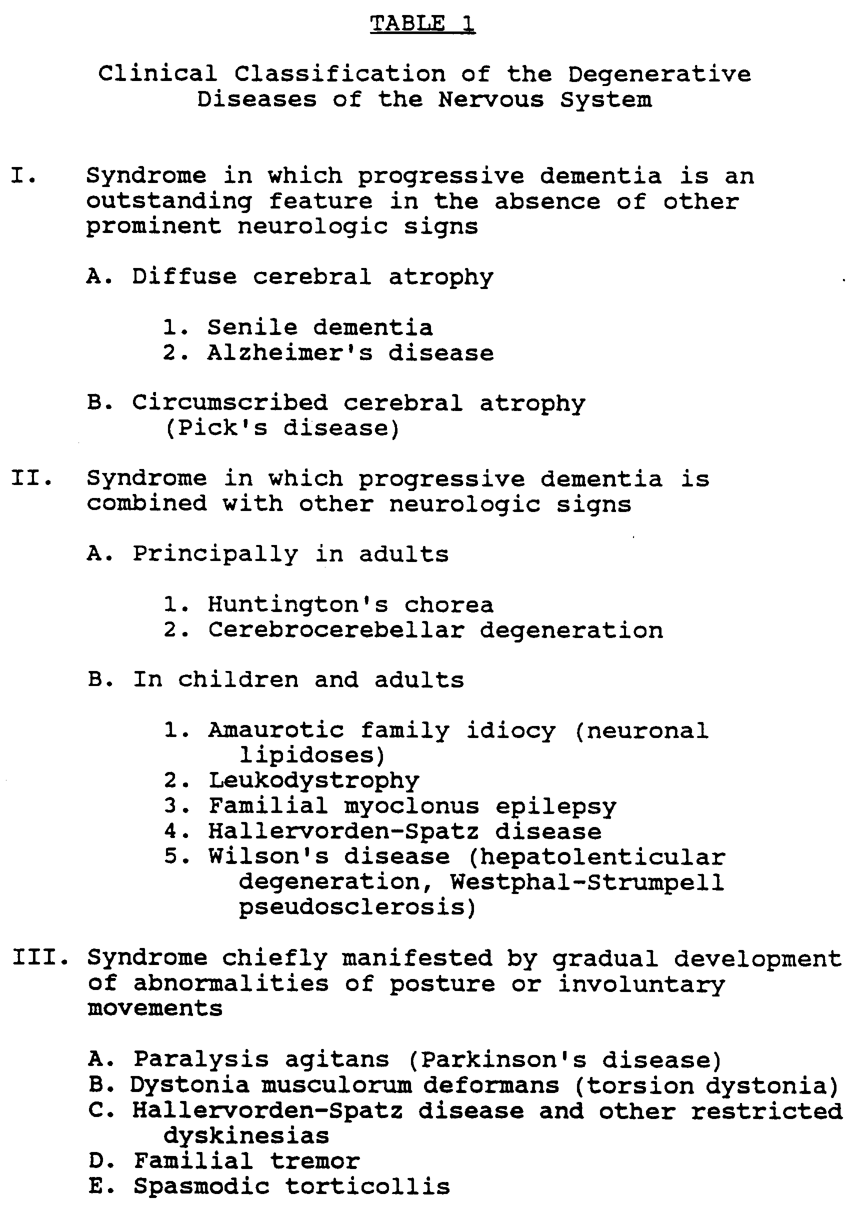

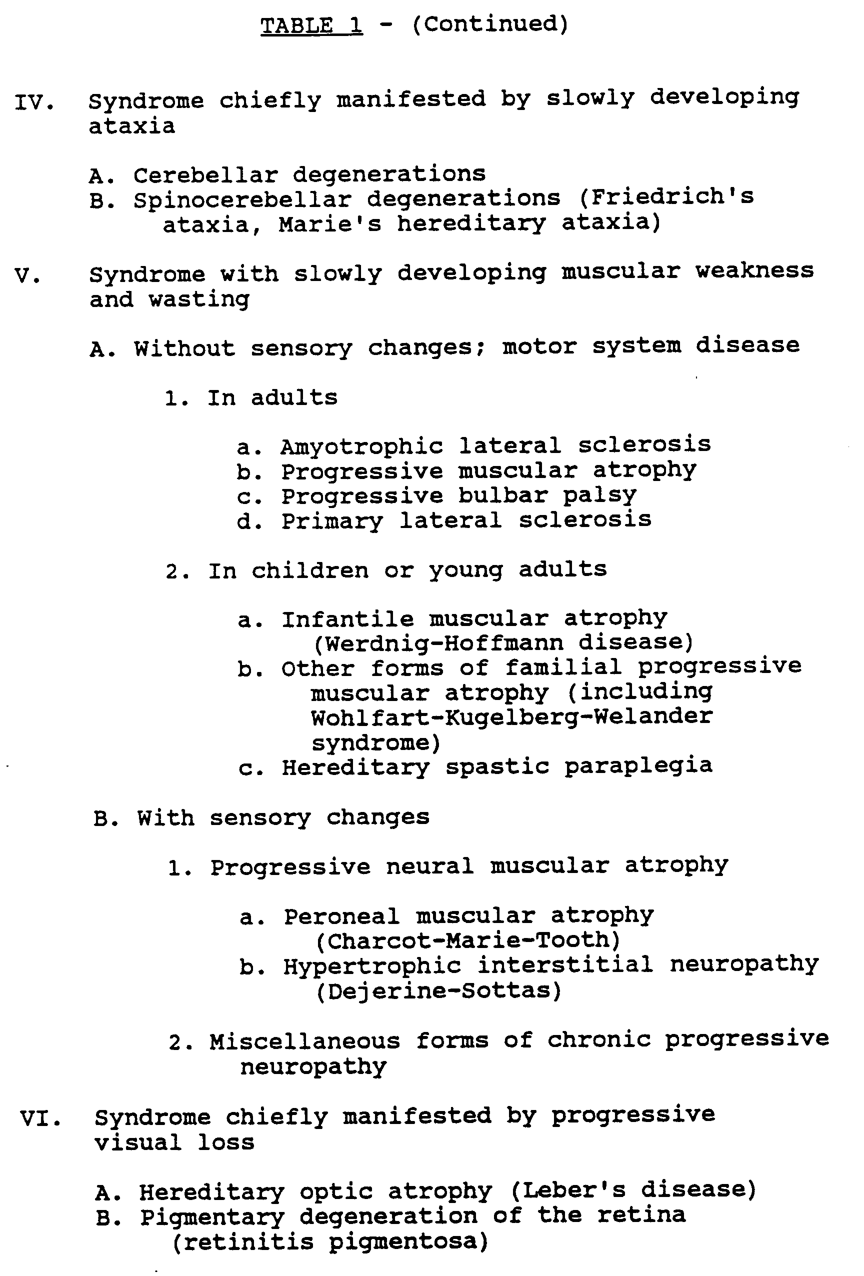

### 2. Parkinson's Disease

Perhaps the disorder the general public is most familiar with is Parkinson's disease, or paralysis agitans. In early stages of the disease, there may be slight disturbances of posture, locomotion, facial expression or speech. The manifestations may be asymmetric, e.g, a slight tremor of the fingers on one hand at rest. The symptoms then become bilateral and the patient tends to assume a stooped posture. Gait disturbances increase and there is a moderate generalized disability. After a number of years the disability, bradykinesia, weakness and rigidity progress to the point of complete invalidism.

Because of the prevalence of Parkinson's disease, it has been the focus of much neurological research. As early as 1953 it was recognized that it was common for there to be a depletion of dopaminergic transmission and a loss of the melanin-containing cells of the substantia nigra. It is not fully clear whether the changes are the result of "demelanination" by cells or actual cell death.

Current therapy for Parkinsonism is the oral administration of levodopa (L-dopa), which is 3-(3,4-dihydroxyphenyl)-L-alanine. Because L-dopa is a precursor of epinephrine and melanin there are certain contraindications. Apparently levodopa can exacerbate malignant melanomas or other skin lesions and can have untoward effects in patients with cardiovascular or pulmonary disease, asthma, or renal, hepatic or endocrine disease.

The deficiency of dopamine synthesis that characterizes Parkinsonism prompted the notion of transplanting dopamine neurons, particularly those of the adrenal medulla, into the brain as replacement therapy. Following successful transplants and alleviation of symptoms in the rotational rat model and in primates with induced lesions, the first transplants of fetal adrenal medulla were made to the striatum in two patients with severe Parkinsonism. Some rewarding effects were registered. Additional successful cases have been reported in the literature. Nevertheless, it is a complicated procedure which requires fetal donor tissue, and there have been a few unexplained deaths in those same studies.

### 3. Alzheimer's Disease

Alzheimer's Disease (AD) generally presents a clinical picture of gradual loss of intellectual capabilities. The incidence of AD in a number of surveys averages between four and five percent of the U.S. population. This translates to approximately 1.3 million cases of severe AD and an additional 2.8 million patients with mild to moderate impairment. The diagnosis of AD is complicated by the lack of a specific clinical marker. Currently a physician must depend on longitudinal observation for the gradual manifestation of the typical neuropathological features, and the support of a diffusely slow electroencephalogram, reduced cerebral blood flow and particular patterns on positron emission tomographic scanning.

Post-mortem examination of the brain shows a generalized atrophy. There are extensive histologic changes in AD dominated by the presence of intracellular amyloid plaques and neurofibrillary tangles. Plaques and tangles are rare, however, in the basal ganglia and substantia nigra. Many specimens from AD patients demonstrate a loss of pigmentation in the area of the locus ceruleus, which is a major source of noradrenergic synthesis in the brain. Proposed treatments for Alzheimer's disease include the administration of memory-enhancing compounds such as those described in U.S. Patent 4,752,610, as well as the administration of substances such as gangliosides and peptide growth factors which aid the regeneration of injured nerve cells (Terry, R.D. et al., Ann.Neurol. 14, 497 (1983)).

### 4. Schizophrenia and Other Diseases

Dopaminogenic neuronal activity may be abnormal in cases of schizophrenia. There is a reduction in fresh volume of substantia nigra in brains of schizophrenics with the majority of that due to a reduction of cell body volume in the medial portions of that region. Nevertheless, the reduction by cells is not as contributory to the fresh volume loss as is reduction of the neuropil. It is unknown whether those observations have a bearing on the hypothesis that dopamine neurons are overactive in schizophrenia.

Human diseases of the basal ganglia result in hyperkinetic or hypokinetic activity. For example, progressive familial myoclonic epilepsy (Unver-Richt-Lundberg-Lafora disease) is characterized by first generalized convulsive seizures followed by myoclonic jerks of increasing frequency and severity, and progressive dementia. Pathologic investigation reveals atypical cellular architecture in the substantia nigra. In Hallervorden-Spatz disease the patient presents a variable clinical picture that includes abnormalities of posture and muscle tone, involuntary movements and progressive dementia.

### 5. Retinitis Pigmentosa (RP)

Because the eye is an ectodermal derivative, that organ, like the brain, contains pigmented cells. Melanocytes are contained in the choroid, which is the structure that supports the multilayered, photosensitive retina. The outermost layer is comprised of pigmented epithelial cells. Those layers of pigmented cells absorb light that passes through the retina and minimizes interference due to reflection.

RP is an ophthalmologic disease characterized by progressive visual field loss and night blindness. The primary defect is at the level of the photoreceptor and pigmented cells of the retina. Currently, there is no known therapy for RP except for cases of vitamin A deficiency and removal of cataracts. Numerous low vision aids such as various magnifiers, telescopes and image intensifiers are available as supportive therapy.

### C. Melanin

For the purposes of the present description, melanins are defined and classified as in the book entitled "Melanins," by R.A. Nicolaus, published in 1968 by Hermann, 115, Boulevard Saint-Germain, Paris, France, which work in its entirety is incorporated herein by reference. As defined by Nicolaus, melanins constitute a class of pigments which are widespread in the animal and vegetable kingdoms While the name "melanin" in Greek means black, not all melanins as pigments are black but may vary from brown to yellow. Melanins are high molecular weight, amorphous polymers of indole quinone. Mammalian colors are determined chiefly by two types, eumelanins and phaeomelanins. Eumelanins are derived from the precursor tyrosine and are generally insoluble and black or brown in color. Phaeomelanins have as their precursors tyrosine and cystine and are generally alkali-soluble and lighter in color. Allomelanins ("allo" meaning other) are formed from nitrogen-free precursors, primarily catechol and 1,8-dihydroxynaphthalene (see The Merck Index, Tenth Edition, page 827, item 5629, Melanins). Quinones are the usual intermediates in allomelanin synthesis. The synthesis of melanins occurs in nature as well as being produced synthetically. A further group of low molecular weight yellow, red and violet pigments is known as trichochromes. The trichochromes are usually classified with the melanins, since they serve as pigments and are derived from the oxidation of tyrosine.

The biosynthetic pathway by which melanin is produced is shown below as reported by Hearing, V.J. et al., Int. J. Biochem. 19, 1141 (1987).

Melanin is formed by hydroxylation and decarboxylation of the amino acids phenylalanine and tyrosine. In one possible anabolic pathway, tyrosine is hydroxylated to form the catecholamine dopa, which is 3,4-dihydroxyphenylalanine, then the diol is oxidized to form the diketone 3,4-dioxyphenylalanine (also known as dopaquinone). The dopaquinone is cyclized to form 5,6-indolequinones, and it is the polymerization of those indolequinones that produces melanin. There are alternative pathways for melanin production. However, in each of those alternatives an understanding of the mechanisms in the final steps remains elusive.

One pathway for melanin production involves the use of the neurotransmitters epinephrine (adrenaline) and norepinephrine (noradrenaline). Epinephrine is oxidized to form adrenochrome, then adrenolutin is produced and finally melanin. But melanin production is more intimately involved with the neural system because tyrosine and phenylalanine are also the precursors for the neurotransmitters epinephrine, norepinephrine and dopamine.

It is not uncommon for metabolic pathways such as these to be intimately involved, for it is a hallmark of "biological economy" that characterizes life processes. Thus, one amino acid building block such as phenylalanine can be used in a number of ways. Similarly, any one of the intermediates in a pathway such as dopamine can serve as starting material for an end product. Catabolism of the end product or intermediates ultimately produces the same building blocks for reconstruction at a later time, or produces unusable catabolites or detoxifies harmful intermediates for removal. Because those pathways are fully integrated, it is common for the end products such as melanin or epinephrine to serve as regulators for the pathway. That phenomenon is known as feedback inhibition. Thus, melanin could inhibit one of the enzymes early in the melanin biosynthetic pathway such as tyrosine hydroxylase. In that way, when melanin concentration is low, tyrosine hydroxylase activity is high and a large amount of tyrosine is converted into dopa for eventual production of melanin. When there is sufficient melanin, tyrosine hydroxylase activity is low and less melanin is produced. That scheme of regulatory economy is typical of metabolism, as is most noted in the endocrine system, of which the neurotransmitters are a part.

The metabolic pathway machinery for the production of products such as melanin and epinephrine from the amino acid building blocks, although likely to be present in all cells, finds maximal presence in those cells that have a high demand for those products, as in the brain. Brain cells have high levels of tyrosine hydroxylase because there is high demand for dopamine, for example. The substantia nigra, that region of the brain where cells are highly pigmented because of the concentration of melanin, is noted for cells with high levels of tyrosinase. In fact, if one performs immunohistochemical analyses of brain sections using an anti-tyrosine hydroxylase antibody, the substantia nigra would be a region of the brain heavily labelled. Because of the intimate relationship between melanin and dopamine, it is not unexpected that the substantia nigra and its pigmented cells have high levels of tyrosine hydroxylase.

Melanin can be prepared synthetically or isolated from natural sources. Natural sources include beef eyes, squid, hair, bacteria such as Streptococcus antibioticus, and brain, among others. Melanins can be prepared synthetically, as described by Froncisz, W. et al., Arch.Biochem.Biophys. 202, 289 (1980) and Lyden, A. et al., Arch.Int.Pharmacodyn. 259, 230 (1982), among others.

Since melanins are polymers of indole quinones, they are polar molecules with exposed amino, keto and carboxyl radicals. The presence of these charged groups allows melanin to act as an effective ionic sponge or chelator. A variety of drugs such as chloroquine and chlorpromazine have a high affinity for melanin (Larson, B. et al., Biochem.Pharmac. 28, 1181 (1979)). Further, there is a high uptake by melanin of serotonin, and moderate uptake of dopamine, noradrenaline and adrenaline, while L-dopa and L-tyrosine have no affinity for melanin (Lindquist, N.G., Acta Radiol.Suppl. 325, 67 (1973)). As mentioned earlier, melanin also has a high affinity for the neurotoxic parkinsonism drug MPTP. High concentrations of MPTP can be found in the substantia nigra and locus cereuleus of animals and patients that have been exposed to the neurotoxin (Snyder, S.H. et al., Neurology 36, 250 (1986)).

Melanin has also been used as a chelator for uranium (Takashi, S. et al., J.Chem.Technol.Biotechnol. 40, 133 (1987)) and as a sorbent for clarifying and stabilizing wine (USSR 924,098).

Melanin has additional anti-toxin characteristics as a free radical scavenger or oxygen scavenger, and as such can serve as a terminator of free radical chain reactions. As a free radical scavenger, melanin may play an important role in preserving cells from the toxic effects of O₂⁻. Geremia, C. et al., Comp. Biochem. Physiol. 79B, 67 (1984).

Melanin has many other interesting properties such as ultraviolet absorption, which has been utilized to prepare optical lenses (U.S. 4,698,374) as well as cosmetic creams (Jap. 49-071149). Melanin has both semiconductor (Culp, C.J. et al., J.Appl.Phys. 46, 3658 (1975)) and superconductor (Cope, F.W., Physiol.Chem. Phys. 10, 233 (1978)) properties.

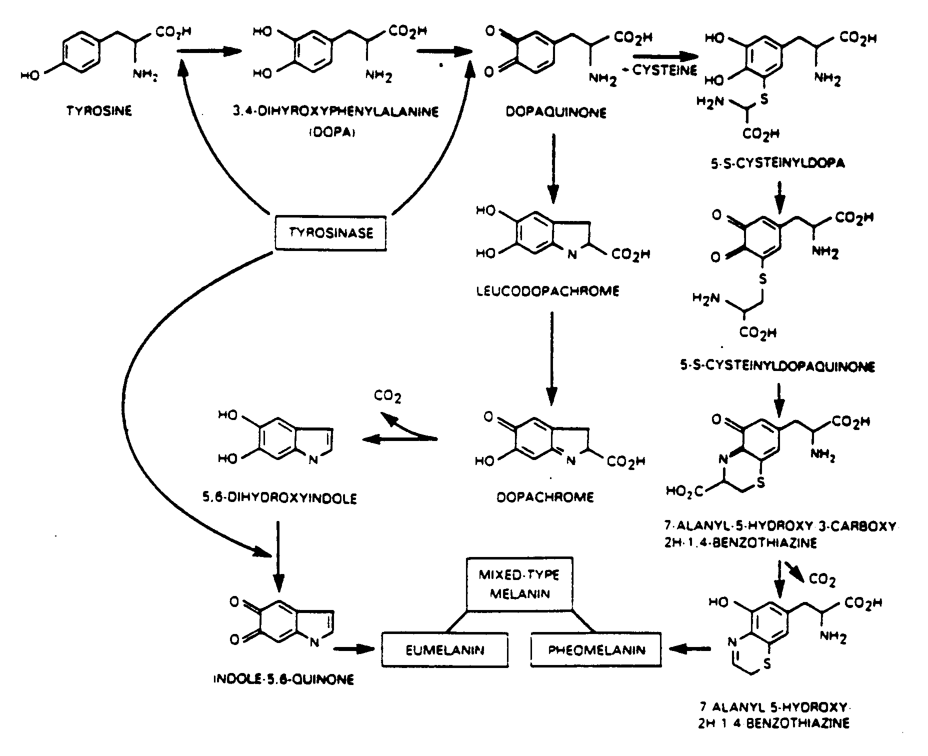

### D. Tyrosinase

The enzyme, tyrosinase, plays a key role in the synthesis of melanin and its derivatives. In mammals, tyrosinase is a glycosylated enzyme found in melanocytes.

It has been theorized that tyrosinase functions by means of separate catalytic sites; one site for tyrosinase hydroxylase activity, another site for dopa oxidase activity, and a third independent site for dopa as a cofactor. Hearing, V.J. et al., Biochem. J., 157 549 (1976). Tyrosinase may also play a role in catalyzing the oxidation of 5,6-dihydroxyindole to indole-5,6-quinone. Korner, A.M. et al., Science 217, 1163 (1982). In vivo, mammalia tyrosinase undergoes extensive modification. When initially synthesized, tyrosinase has an apparent molecular weight of about 55,000. Glycosylation of the enzyme occurs as it is transferred through the Golgi complex and delivered to the melanocytes. Imokawa, G. et al., J. Invest. Derm., 85, 165 (1985). During this modification of tyrosinase, sialic acid and 4 mol of asparagine-linked carbohydrate chains (containing mannose, glucosamine, galactose and fucose) are added to each mole of tyrosinase. Ferrini, V. et al., Int. J. Biochem. 19, 229 (1987). The glycosylated tyrosinase has an apparent molecular weight of about 70,000. Laskin, J.D. et al., J. Biol. Chem. 261, 16626 (1986).

The glycosylated tyrosinase is delivered to the melanocytes by coated vesicles. In the melanocytes, the tyrosinase is membrane bound and aggregates into a high molecular weight form. In vivo, tyrosinase is under active metabolic control involving an active degradation system which results in a biological half-like of about ten hours. Jimenez, M. et al., Fed. Proc. Fodn. Am. Socs. Exp. Biol. 45, 1714 (1986).

### E. Tyrosinase Gene

The gene for human tyrosinase has been isolated, sequenced and cloned (PCT application WO 88/02372, published April 7, 1988). The cloned gene encodes a polypeptide of 548 amino acids with a molecular weight of 62,160, excluding a hydrophobic signal peptide. It is suggested in the PCT application that the tyrosinase gene is involved in melanin biosynthesis in addition to tyrosinase.

The gene for Streptomyces glaucescens tyrosinase has also been isolated and sequenced (Huber, M. et al., Biochemistry 24, 6038 (1985)). Nearly all of the codons used end in either G or C, and the overall G + C content of the gene is 71.4%. Id.

In order to isolate the S. glaucescens tyrosinase gene, the KpnI fragment of plasmid pMEA4 containing the S. glaucescens gene (Hintermann, G. et al., Mol. Gen. Genet. 200, 422 (1985)) is cloned into the PvuII site of pBR322 with KpnI linkers (P-L Biochemicals). Two resulting plasmids (pMEA6 and pMEA7) contain the tyrosinase gene in opposite directions. (Huber, M. et al., supra). Plasmid DNA is then isolated by conventional techniques such as those described by Maniatis, T. et al., Molecular Cloning, Cold Spring Harbor Laboratory, Cold Spring Harbor (1982).

Restriction endonucleases are then used according to the suppliers' instructions (Boehringer, Amsterdam) to perform digestions, and the fragments are recovered by low-melting agarose gels as described by Weislander, L., Anal. Biochem. 98, 305 (1979). The nucleotide sequences are then determined using the methods of Maxam, A.M. et al., Methods. Enzymol. 65, 499 (1980).

### F. Melanin Concentrating Hormone

Melanin concentrating hormone (MCH) is a peptide which has been isolated from fish pituitary gland, characterized and synthesized (Kawauchi, H. et al., Nature 305, 321 (1983)). MCH has also been localized by immunohistochemistry in the brain and pituitary gland of salmons, frogs and rats (Baker, B.J. et al., Gen. Comp. Endocrinol. 50, 1423 (1983), Naito, N. et al., Neurosci. Lett. 70, 81 (1986), Skotfitsch, G. et al., Proc. Natl. Acad. Sci. USA 83, 1528 (1986) and Zamir, N. et al., Brain Research 373, 240 (1986)).

A mammalian MCH-like substance has been recognized by salmon MCH-directed antiserum in radioimmunoassay and immunohistochemistry (Zamir, N. et al., Proc. Natl. Acad. Sci. USA, supra). This mammalian MCH has been diluted in parallel with synthetic MCH, but exhibits distinct chromatographic properties on both RP-HPLC and gel chromatography. Id. The persistence of this mammalian MCH in the mammalian hypothalamoneurohypophyseal system suggests a role in posterior pituitary function, such as the regulation of food and water intake. Id.

Other functions of this mammalian MCH peptide have also been suggested. Due to the identification of MCH fibres in the human median eminence and pituitary stalk, it has been suggested that the peptide causes the aggregation or concentration of melanin in cells of the central nervous system and may be involved in the regulation of anterior pituitary function (Pelletier, G. et al., Brain Research 423, 247 (1987)). Furthermore, Sekiya, K. et al. in Neuroscience 25, 925 (1988) suggest that MCH may act as a neurotransmitter and/or neuromodulator in the central nervous system or may regulate pituitary portal-blood system and/or the neurosecretory system in mammals.

The present invention is directed to the use of an active substance which cause an increase in the concentration of melanin in the tissue, e.g. in the patient's central nervous system (CNS), said active substance is selected from melanin, melanin derivatives in combination with melanin, tyrosinase, tyrosinase gene or melanin concentrating hormone, melanin-concentrating hormone (MCH), tyrosinase, tyrosinase gene and combinations thereof in the manufacture of a medicament to treat degenerative diseases of the nervous system. These diseases include those of tissues which have lost melanin (melanin deficency) and which share a common embryological basis as the nervous system. More specifically, the use of melanin or a melanin derivative in the above mentioned combination replaces lost melanin in the treatment of said diseases which exhibit a decrease in the production of melanin and/or exhibit an increase in the catabolism or excretion of melanin. Alternatively, the use of MCH causes the concentration of available melanin in particular areas of the CNS, and the administration of tyrosinase or tyrosinase gene allows the patient's body to produce more melanin by increasing the conversion of melanin precursors to melanin. The diseases include Parkinson's disease, Alzheimer's disease, retinitis pigmentosa, depression, schizophrenia and other diseases such as those listed in Table 1 above.

The present invention is also useful for assisting the recovery of neurons in a mammal having neuron injury by administering an effective amount of an active substance which causes an increased concentration of melanin in the neuron to aid in nerve recovery. Melanin or a melanin derivative in the above mentioned combination can be administered to accomplish this result. Alternatively, the melanin necessary to aid nerve recovery may be concentrated in the CNS by administration of MCH, or may be produced in the patient's body by administering tyrosinase which catalyzes naturally occurring melanin precursors to melanin. Furthermore, the administration of tyrosinase gene causes the production of tyrosinase in the patient's body, thereby catalyzing the conversion of the naturally occurring melanin precursors to melanin. The present invention is further useful in protecting a mammal from a neurodegenerative disease, or the adverse effects of neurodegenerative disease-causing substances.

In order to provide a clear and consistent understanding of the specification and claims, including the scope given to such terms, the following definitions are provided:

Administration: The application or delivery of a drug to a mammal in need of the drug. This term is intended to include any means of administration which accomplishes the application or delivery of the drug (i.e., topical, oral, aerosol, suppository, intravenous, intramuscular, injection, e.g., into the cerebrospinal fluid or other parts of the nervous system, peritoneally and the like). The term is also intended to include any means necessary to accomplish such administration, such as a sugar loading procedure to enable a drug to cross the blood-brain barrier. The term is further intended to include the in vivo production of a drug or aggregation of a drug moderated by another substance such as an enzyme (tyrosinase) or enzyme gene (tyrosinase gene) to moderate production of a drug (melanin), or a concentrating hormone (MCH) to moderate drug (melanin) concentration.

Blood-Brain Barrier: The blood-brain barrier is made up of brain microvessel endothelial cells characterized by tight intercellular junctions, minimal pinocytic activity, and the absence of fenestra. These characteristics endow these cells with the ability to restrict passage of most small polar blood-borne molecules (e.g., neurotransmitter catecholamines, small peptides) and macromolecules (e.g., proteins) from the cerebrovascular circulation to the brain. The blood-brain barrier contains highly active enzyme systems as well, which further enhance the already very effective protective function. It is recognized that transport of molecules to the brain is not determined solely by molecular size but by the permeabilities governed by specific chemical characteristics of the permeating substance. Thus, besides molecular size and lipophilicity, the affinity of the substances to various blood proteins, specific enzymes in the blood, or the blood-brain barrier will considerably influence the amount of the drug reaching the brain.

Common Embryological Basis: This term is intended to include all tissues which are derived from the same germinal layer, specifically the ectoderm layer, which forms during the gastrulation stage of embryogenesis. Such tissues include, but are not limited to, brain, epithelium, adrenal medula, spinal chord, retina, ganglia and the like.

Degenerative Diseases of the Nervous System: This term is intended to include any of the diseases referred to in Table 1 as well as other brain disturbances including, but not limited to, depression, dementia and schizophrenia. This term is used interchangeably with the terms "diseases with a neurological dysfunction or disorder" or "neurodegenerative diseases," which are intended to have the same meaning.

Melanin: A high molecular weight amorphous polymer of indole quinone, including eumelanins, phaeomelanins, neuromelanins and allomelanins. This term is also intended to include trichochromes. When melanin is used hereafter, it is intended to include both melanin and melanin derivatives unless the context dictates otherwise.

Melanin Deficiency: This term is intended to refer to a condition in diseased tissue in which melanin is absent, present in a lower amount when compared to normal tissue, or functionally non-active. The deficiency may be caused by a decrease in the synthesis of melanin and/or an increase in the catabolism or excretion of melanin. The melanin may be functionally non-active as the result of a substance binding to it which destroys the melanin's activity.

Melanin Derivative: This term is intended to include any derivative of melanin which is capable of being converted in tissue to either melanin or a substance having melanin activity. An example of a melanin derivative is melanin attached to a dihydrotrigonelline carrier such as described in Bodor, N., Ann. N.Y. Acad. Sci. 507, 289 (1987) to enable the melanin to cross the blood-brain barrier.

Neurodegenerative Disease-Causing Substance: Any substance which can cause a neurodegenerative disease in a mammal. Examples of such substances include N-methyl-4-phenyl-1,2,3,6-tetrahydropyridine (MPTP), 1-methyl-4-phenylpyridine (MPP⁺) and manganese dust for Parkinson's disease; quinolinic acid for Huntington's chorea; and β-N-methylamino-L-alanine for amyotrophic lateral sclerosis, Parkinson's disease and Alzheimer's disease.

Tyrosinase: An enzyme which, in mammals, catalyzes: (a) the hydroxylation of tyrosine to dopa (3, 4-dihydroxyphenylalanine); (b) the oxidation of dopa to dopaquinone; and (c) may catalyze the oxidation of 5,6-dihydroxyindole to indole-5,6-quinone. All of these reactions which are catalyzed by tyrosinase take place in the biosynthetic pathway which produces melanin. Tyrosinase is most commonly found in a glycosylated form in vivo.

In order to develop therapy programs for any disease, it is useful to identify (a) potential causes of the disease, in an attempt to avoid them; (b) potential manifestations of the disease, in an attempt to identify aspects of the disease which may be treated, and (C) drugs which are similar to known therapeutic drugs. Little is known as to the cause-and-effect relationship in the neurodegenerative diseases. One problem in these diseases is that few animal models exist which can be utilized to gain the necessary understanding of each disease and its treatment.

Probably the most studied disease in terms of brain pathology has been Parkinson's disease. It is well known that substantial changes occur within the substantia nigra of patients suffering from Parkinsonism. As previously discussed, the substantia nigra is one of the most heavily pigmented areas of the brain and consequently contains significant amounts of melanin. It has been demonstrated that cell death in the substantia nigra in Parkinson's disease is related to a loss of melanin in the neurons of the substantia nigra (Mann et al., supra; Hirsch, E. et al., Nature 334, 345 (1988)). Furthermore, it has been established that MPTP, which can cause Parkinson's disease, binds to neuromelanin (D'Amato et al. (1986), supra) and is concentrated in the substantia nigra and locus cereuleus (Snyder et al., supra).

The common factor of for example Parkinson's disease, Alzheimer's disease, progressive familial myoclonic epilepsy, Hallervorden-Spatz disease, and retinitis pigmentosa is that a tissue which is highly pigmented, i.e., one which contains melanin, is involved in the disease. In almost every instance, there is a decreased melanin content, i.e., a loss of pigment, which may lead to cell death. As described further below, applicant has discovered that treatment of neurodegenerative diseases with melanin can ameliorate the primary neurological symptoms of the disease.

An active substance such as melanin can be used in the manufacture of a medicament to treat neurodegenerative diseases. As discussed above, the loss of melanin can be seen in many neurodegenerative diseases. For example, the retina suffers a loss of pigmented cells in retinitis pigmentosa. In Alzheimer's disease there is a generalized atrophy and a loss of pigment, i.e., melanin, in the area of the locus ceruleus, which is a major source of noradrenergic synthesis in the brain. A reduction in fresh volume of the substantia nigra, especially of the neuropil, has been seen in schizophrenics. A typical cellular architecture also exists in Unver-Richt-Lundberg-Lafora disease.

As discussed above, it has been demonstrated that cell death in the substantia nigra in Parkinson's disease is related to a loss of melanin in the neurons of the substantia nigra (Mann et al., supra; Hirsch, E. et al., Nature 334, 345 (1988)).

It has now been found that the administration of melanin to a mammal having a neurodegenerative disease, is capable of ameliorating the primary neurological symptoms of the neurodegenerative disease which is treated. Similar improvement in overall functional ability is also improved. Furthermore, secondary motor manifestations of the neurodegenerative diseases are also proportionately improved upon administration of melanin. The melanin can be administered by any means which will insure that it reaches the desired tissue. In many instances, the administration will require mechanisms for crossing the blood-brain barrier. Several mechanisms are described below and others are known in the art. Since the treatment of the disease will require many separate doses of melanin, some mechanisms will be more preferred than others. Suitable doses for this purpose are from about 0.5 to about 150 mg/kg/day and preferably from about 1 to about 50 mg/kg/day of the active ingredient. Proper doses are determined as described below.

Melanin can in particular be used for ameliorating Alzheimer's disease since it is capable of aiding the recovery of injured neurons (discussed in further detail below). Suitable doses for this purpose are as described above, and the optimal dose is determined as described below.

An alternative method is to enhance the in vivo production of melanin by administering tyrosinase to the effected patient. Tyrosinase catalyzes at least two, and possibly three, of the reactions in the biosynthetic pathway which produces melanin.

Naturally, occurring tyrosine in the human body is hydroxylated to 3,4-dihydroxyphenylalanine (dopa), and the hydroxylation is catalyzed by tyrosinase. Tyrosinase also catalyzes the subsequent oxidation of dopa to dopaquinone. The dopaquinone is a precursor for two separate biosynthetic pathways for the production of melanin. Therefore, both tyrosinase-catalyzed reactions which lead to the production of dopaquinone (the hydroxylation of tyrosine to dopa and the oxidation of dopa to dopaquinone) are important reactions in the human body's production of melanin.

One pathway from dopaquinone to melanin involves a ring closure and hydrogenation of dopaquinone to produce leucodopachrome. This is followed by partial oxidation of leucodopachrome to dopachrome, and decarboxylation and hydroxylation of the dopachrome to 5,6-dihydroxyindole. The 5,6-dihydroxyindole is then oxidized to indole-5,6-quinone, and it is at this step that tyrosinase is again believed to serve as a catalyst. Korner, A.M. et al., Science 217, 1163 (1982). Tyrosinase is believed to catalyze this oxidation reaction. The indole-5,6-quinone is then converted to melanin or eumelanin.

The other pathway from dopaquinone to melanin involves the addition of cysteine to dopaquinone to produce 5-S-cysteinyldopa, followed by the oxidation of 5-S-cysteinyldopa to 5-S-cysteinyldopaquinone. A ring closure of the 5-S-cysteinyldopaquinone then yields 7-alanyl-5-hydroxy-3-carboxy-2H-1,4-benzothiazine which is subsequently decarboxylated to yield 7-alanyl-5-hydroxy-2H-1,4-benzothiazine. At this point, the 7-alanyl-5-hydroxy-2H-1,4-benzothiazine is converted to melanin and pheomelanin. Tyrosinase does not play any additional role in this melanin production pathway.

It has now been found that the administration of tyrosinase to a mammal having a neurodegenerative diseases is capable of ameliorating the primary neurological symptoms of the neurodegenerative disease which is treated. Similar improvement in overall functional ability is also improved. Furthermore, secondary motor manifestations of the neurodegenerative diseases are also proportionately improved upon administration of tyrosinase. These improvements are believed to be due to the increased in vivo production of melanin brought about by the increased tyrosinase-mediated catalysis of reactions along the biosynthetic pathway responsible for the production of melanin.

The tyrosinase can be administered as described above by any means which will insure that it reaches the desired tissue. The amount of tyrosinase administered must be sufficient to catalyze the melanin-producing reactions such that sufficient melanin is produced to alleviate the disease symptoms. Proper doses are determined as described below.

Tyrosinase can in particular be used for ameliorating the symptoms of Alzheimer's disease since it increases the production of melanin in vivo, and melanin is capable of aiding the recovery of injured neurons (discussed in further detail below). Suitable doses for this purpose are as described above, and the optimal dose is determined as described below.

Another method by which the in vivo production of melanin may be enhanced is by the administration the tyrosinase gene to the effected patient. After administration, the tyrosinase gene transfects susceptible mammalian cells and tyrosinase is produced. The tyrosinase, in turn, catalyzes the production of melanin from naturally occurring melanin precursors as explained above.

The most common method by which tyrosinase gene is introduced into the mammalian system is by its incorporation into a defective herpes simplex virus 1 (HSV-1) vector. Particularly, the defective HSV-1 vector, pHSVlac, developed by Geller et al., Science 241, 1667 (1988) is especially useful for this purpose. This vector is useful for transneuronally transporting genes from peripheral neurons to the primary target cells in the brain (Ugolini et al., Science 243, 89 (1989)). The amount of tyrosinase gene administered must be sufficient to transfect susceptible mammalian cells so that tyrosinase is produced therefrom.

With a medicament containing melanin-concentrating hormone the concentration of naturally occurring melanin at the target cells in the central nervous system can be increased Commonly, a combination of MCH and tyrosinase or tyrosinase gene is administered as an effective combination for the treatment of the neurodegenerative disease. The tyrosinase or tyrosinase gene causes an increased melanin production, and the MCH induces the aggregation of melanin in the target cells and tissues.

The active substance such as melanin can be used to prevent degenerative diseases of the nervous system which are caused by exposure of a mammal to toxic agents which cause such neurodegenerative diseases. Toxic agents which are known to cause neurodegenerative diseases include N-methyl-4-phenyl-1,2,3,6-tetrahydropyridine (MPTP) and 1-methyl-4-phenylpyridine (MPP⁺) and manganese dust for Parkinson's disease; quinolinic acid for Huntington's chorea; β-N-methyl-amino-L-alanine for amyotrophic lateral sclerosis, Parkinson's disease and Alzheimer's disease; and aluminum has been implicated in Alzheimer's disease. In addition to these agents, the toxic metabolite of MPTP, MPP⁺, has been field-tested as a herbicide under the name Cyperquat. The well-known herbicide Paraquat chemically resembles MPP⁺. Cyperquat and Paraquat are pyridine derivatives. Many analogs of MPTP exist in the environment and could also be involved in idiopathic parkinsonism. One of the MPTP analogs, 4-phenylpyridine, a constituent of peppermint and spearmint tea, was toxic to catecholamine neurons in vitro (Snyder et al., supra). Melanin can also be used to prevent the adverse effects caused by toxins which are absorbed, inhaled or ingested by a mammal. In addition to the toxins discussed above, other toxins include, but are not limited to, metals, metal-containing compounds, radioisotopes and radioactive compounds, including radioactively labelled therapeutics and diagnostics. Metals include, but are not limited to, aluminum, lead and manganese. Melanin is especially useful as a chelating agent to lower or eliminate aluminum agents.

Melanin has been found to be able to bind MPTP as well as MPP⁺. Administration of melanin can thus effectively bind MPTP, MPP⁺ and other neurodegenerative disease-causing substances before the substances reach the tissue (especially brain tissue) they damage. The melanin can be administered by any means, but for present purposes it is preferred to administer it orally, by inhalation or suppositories. Suitable doses for this purpose are from about 0.5 to about 100 mg/kg, and preferably from about 1 to about 5 mg/kg of the active ingredient. This aspect of the invention is shown in Examples 1 and 2 below.

Alternatively, the administration of tyrosinase can increase the production of melanin in vivo, thereby causing the binding of neurodegenerative disease-causing substances before the substances reach the tissue (especially brain tissue) which they damage. The tyrosinase can be administered by any means, but for present purposes it is preferred to administer it orally, by inhalation or by suppositories. As in the case of treating melanin deficiency diseases, the amount of tyrosinase administered must be sufficient to catalyze the melanin producing reactions such that sufficient melanin is produced to alleviate the disease symptoms.

As is also the case with treatment of melanin deficiency diseases, another method by which the in vivo production of melanin may be enhanced by the administration of the tyrosinase gene to the effected patient. After administration, the tyrosinase gene transfects susceptible mammalian cells and tyrosinase is produced. The tyrosinase, in turn, catalyzes the production of melanin from naturally occurring melanin precursors as explained above.

The most common method by which tyrosinase gene is introduced into the mammalian system is by its incorporation into a defective herpes simplex virus 1 (HSV-1) vector. Particularly, the defective HSV-1 vector, pHSVlac, developed by Geller et al., Science 241, 1667 (1988) is especially useful for this purpose as explained above. The amount of tyrosinase gene administered must be sufficient to transfect susceptible mammalian cells so that tyrosinase is produced therefrom.

A further aspect of prophylaxis is to increase the concentration of naturally occurring melanin at the target cells in the central nervous system by the administration of melanin-concentrating hormone (MCH). Commonly, a combination of MCH and tyrosinase or tyrosinase gene is administered as an effective combination for the treatment of melanin deficiency diseases. The tyrosinase or tyrosinase gene causes an increased melanin production, and the MCH induces the aggregation of melanin in the target cells and tissues.

The active substance such as melanin can be used to assist in the recovery of injured neurons. The neurons could be injured as a result of direct injury or disease. For example, it is known that MPTP destroys a substantial number of dopaminergic nerve terminals in the striatum of young mature mice, and that after five months there is a substantial, though incomplete, recovery of striated dopamine nerve terminal markers. Ricourte, G.A. et al., Brain Res. 376, 117 (1986). It is also known that melanin is present in all neurons in the form of dark, irregularly shaped granules called Nissl bodies. Nissl bodies are scattered throughout the cytoplasm and occur in dendrites of the larger neurons. They appear to be absent in the axon and axon-hillock. In pathological conditions, there is a partial or complete reduction in the amount of Nissl bodies. For example, it is known that Nissl bodies disappear with nerve injury but reappear upon nerve recovery. Within certain regions of the brain, there are areas of neurons that have high concentrations of Nissl bodies, thereby rendering localized regions black. Examples of these areas include the substantia nigra and locus ceruleus. It is known that Nissl bodies disappear with nerve injury but reappear upon nerve recovery. It has been found that the administration of melanin or a melanin derivative is able to aid in the recovery of neurons by accelerating the time frame for neuron recovery. This aspect of the invention is shown in Example 3 below.

It has also been found that the administration of tyrosinase, tyrosinase gene, MCH or combinations thereof aid in the recovery of neurons. The tyrosinase increases the production of melanin in vivo, and the melanin accelerates the time frame for neuron recovery. The administration of tyrosinase gene and/or MCH aids in neuron recovery by promoting the same reactions described above for treatment and prophylaxis of melanin deficiency diseases.

Pharmaceutical compositions containing the active substance of the present invention (i.e. melanin, melanin derivatives, tyrosinase, tyrosinase gene, MCH and combinations thereof) in intimate admixture with a pharmaceutical carrier can be prepared according to conventional pharmaceutical compounding techniques. The carrier may take a wide variety of forms depending on the form of preparation desired for administration, e.g., intravenous, oral, topical, aerosol, suppository, parenteral or spinal injection. In preparing the compositions in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. For parenterals, the carrier will usually comprise sterile water, though other ingredients, for example, to aid solubility or for preservative purposes, may be included. Injectable suspensions may also be prepared, in which case appropriate liquid carriers, suspending agents, pH adjusting agents, isotonicity adjusting agents and the like may be employed. For topical administration, the carrier may take a wide variety of forms depending on the form of preparation, such as creams, dressings, gels, lotions, ointments or liquids. Aerosols are prepared by dissolving or suspending the active ingredient in a propellant such as ethyl alcohol or in propellant and solvent phase. Suppositories are prepared by mixing the active ingredient with a lipid vehicle such as theobroma oil, cacao butter, glycerin, gelatin, or polyoxyethylene glycols. The pharmaceutical compositions for topical or aerosol form will generally contain from about 1% by weight to about 40% by weight, depending on the particular form employed.

There are unique considerations in the treatment of central nervous system dysfunction. Unlike other tissues, brain tissue is not redundant. It is highly differentiated, compartmentalized, and cannot be replaced. Thus, neuropharmaceutics must be found non-toxic to normal tissue. The real problem, however, has been to find the most efficacious route of circumventing the blood-brain barrier. One way to bypass the barrier is by intracerebrospinal fluid administration by lumbar puncture or by the intraventricular route. Catheterization using the Ommaya reservoir is used, but logistics dictate that to be a last-resort method.

Because the barrier is selective, some drugs can be administered orally. Certain lipophilic chemicals or moieties that mimic the neural amino acids can bypass the barrier by mere diffusion or by transport via the energy dependent membrane-bound carrier, respectively.

An example of a drug administered intrathecally is methotrexate, an antineoplastic agent, in the treatment of meningeal leukemia. The sodium salt of methotrexate is administered in solution in doses of 12 mg per square meter of body surface or in an empirical dose of 15 mg. The drug is given every two to five days until the cell count of the cerebrospinal fluid returns to normal. L-dopa can be used to compensate for the depletion of dopamine that occurs in parkinsonism because it also passes freely through the blood-brain barrier.

Transient reversible modification of the blood-brain barrier is accomplished in either of two ways --osmotic opening or metrazol opening. The first method is based on increasing capillary permeability by osmotically-induced shrinkage of the endothelial cells which caused widening of the intercellular tight junctions. The osmotic load is generally a hyperosmotic water-soluble agent such as mannitol or arabinose. Briefly, under general anesthesia a transfemoral catheter is introduced into the internal carotid or vertebral artery and 150-300 ml infusion of 25% mannitol is administered at 6-10 mg/sec for 30 seconds. The intravenous infusion of melanin or tyrosinase is begun approximately five to seven minutes before the mannitol infusion and is continued for 15 minutes. The transfemoral catheter is removed and the patient observed for 24-48 hours.

Alternatively, the active agent (melanin or tyrosinase) may be linked to the osmotic agent (mannitol, arabinose, glucose or other sugar moiety), and a single infusion may be used. Conventional techniques may be used to link the active agent and the osmotic agent. The linked agent itself will then cause the osmotically-induced shrinkage of the endothelial cells in order to widen the tight intercellular junctions. The linked agent may be designed such that the active agent (melanin or tyrosinase) is cleaved from the linked agent after the blood brain barrier has been crossed.

In the second method, capillary permeability is increased by eliciting seizure activity using a central nervous stimulant such as pentylenetetrazol. The technique is similar to that of osmotic opening with the replacement of mannitol infusion by parental delivery of the stimulant.

A drug also can be disguised so that is able to cross the blood-brain barrier. One method of accomplishing the disguise is to prepare a redox system as described by Bodor, supra. In this system a derivative of the drug is prepared which is capable of crossing the blood-brain carrier and which is converted in tissue to the drug or to a substance having the activity of the drug. In the case of melanin or tyrosinase, a derivative is prepared by attaching melanin or tyrosinase to a dihydrotrigonelline carrier such as described in Bodor, supra.

A similar method of disguising a drug so that it will cross the blood brain barrier is to create a redox system in which the drug is coupled to a pyridinium carrier as described by Bodor, N. et al., Pharmac. Ther. 19, 337 (1986). Commonly used pyridinium carriers include substituted nicotinic acid and nicotinamide. After coupling, the drug-carrier complex is reduced, yielding a dihydropyridine. The reduced complex is then administered systemically. The reduced complex will cross the blood brain barrier due to its enhanced membrane permeability, and it will also be distributed elsewhere in the body.

At all locations in the body (in the brain as well as elsewhere in the body) the reduced drug-carrier complex will be subject to oxidation. However, the rate of oxidation can be controlled to some extent by selected substitution of the pyridine ring. Following oxidation, the charged drug-carrier complex is rapidly eliminated from the peripheral blood system by renal and/or biliary processes. However, the compound will be retained in the brain due to its size and charge. The cleavage of the drug from the oxidized carrier will also occur in both the brain and the periphery, and if this cleavage occurs at a more rapid rate than the efflux of complex from the brain, a sustained release of the drug in the brain will be achieved. In the case of melanin or tyrosinase, a drug-carrier complex is prepared by coupling the melanin or tyrosinase to nicotinyl chloride as described by Bodor, N. et al., supra.

A further alternative method for delivering melanin or tyrosinase to target areas of the brain is to transport the tyrosinase gene into the brain by means of a defective Herpes simplex virus-1 (HSV-1) vector using a method described by Geller, A.I. et al., Science 241, 1667 (1988). Particularly, the defective HSV-1 vector described by Geller, A.I. et al., supra, is pHSVlac, which contains the Escherichia coli lacZ gene under the control of the HSV-1 immediate early 4/5 promoter.

In order to use this HSV-1 vector in the present invention, the tyrosinase gene (as isolated and identified by Huber, M. et al., Biochemistry 24, 6038 (1985)) is inserted into the defective HSV-1 vector in place of the E. coli lacZ gene using conventional techniques. This new vector containing the tyrosinase gene can then enter the brain where the tyrosinase gene will be replicated and transcribed to produce tyrosinase which in turn will catalyze melanin production in the immediate vicinity of the target cells.

As with most neurologic drugs, there is no established dosage of melanin or tyrosinase. The regimen is determined empirically for each patient. The optimal dose is that which produces maximal improvement with tolerated side effects. For example, an initial dose of 0.5-1.0 gm/day with the total daily dosage increasing in increments not more than 0.75 gm every three to seven days as tolerated, is a recommended regimen. Although the optimal therapeutic dosage should not exceed 8 gm per day, patients may be given more as required. It is worth emphasizing that in both of the above cases, optimal dosage is determined empirically and balances the benefits and adverse side effects.

### Examples

The invention is further illustrated by the following non-limiting examples. Example 1 demonstrates melanin's capability of chelating toxins such as MPTP. Example 2 shows that toxin-induced Parkinson's disease can be prevented if the toxin cannot bind to melanin in the brain. Since administered melanin can chelate toxins, it prevents the toxins from binding to melanin in the brain and causing neurodegenerative diseases. Example 3 demonstrates that melanin can be used to aid neuron recovery. Example 4 shows the use of melanin for the treatment of Parkinson's disease.

### EXAMPLE 1

### Affinity of Melanin for MPTP

1-Methyl-4-phenyl-1,2,5,6-tetrahydropyridine (MPTP) is synthesized in accordance with the method described by Schmidle and Mansfield (1955). Purity and identity are confirmed by thin-layer chromatography and gas chromatography-mass spectrometry.

Melanin from beef eyes is prepared according to Potts, A.M., Ophthalmol. 3, 405 (1964). The pigment is finally suspended in distilled water to a concentration of 10 mg (by dry weight) per ml suspension. It has been found that the melanin content of pigment granules from beef eyes is approximately 50% (Larsson et al., supra), which gives a concentration of 5 mg of pure melanin per ml suspension.

Synthetic dopamine melanin is prepared by autooxidation (Lyden et al., supra). The dopamine melanin suspension (in distilled water) is adjusted to contain 5 mg melanin per ml. Both pigment suspensions are stored at 2°C.

The binding of MPTP to the melanins is analyzed as previously described in detail by Lyden et al., supra. Six and one-half ml of various concentrations of MPTP (5.7 µM - 1.2 mM) are mixed with 0.5 ml aliquot portions of melanin suspension. The reaction mixtures are incubated at room temperature for one hour. Reference samples contain distilled water instead of melanin. The mixtures are then centrifuged at 35,000 x g for 10 minutes and the concentration of free MPTP in supernatants is measured spectrophotometrically at 243 nm after appropriate dilution. The uptake of MPTP on melanin is calculated from the differences in concentrations between the supernatants and the reference samples.

From the obtained data, the classes of binding sites, association constants and the binding capacity of the melanins are estimated according to Scatchard, G. et al., J. Am. Chem. Soc. 79, 12 (1957). As the molecular weight of melanin is unknown, the value for the number of binding sites is expressed as mol per mg melanin. The calculations are based on a melanin content of 2.5 mg per incubation.

MPTP is bound to both isolated beef eye melanin and synthetic dopamine melanin in vitro.

The calculated binding parameters are shown in Table 2. The association constants (K) are expressed as M⁻¹, and the number of binding sites (n), as µ mole per mg melanin.

**TABLE 2**

| Binding Parameters for the Interaction of MPTP with Melanin | | |
|---|---|---|
| Beef-eye melanin | n₁ = 0.09 | K₁ = 2.32 x 10⁵ |
| | n₂ = 0.44 | K₂ = 1.22 x 10³ |
| Dopamine melanin | n₁ = 0.08 | K₁ = 5.82 x 10⁵ |
| | n₂ = 0.05 | K₂ = 1.22 x 10⁴ |
| | n₃ = 0.14 | K₃ = 7.68 x 10² |

Curvilinear Scatchard plots are observed for both melanins, which indicates that more than one binding class must be implicated. The data for the binding of MPTP to beef eye melanin could be fitted by the assumption of two classes of binding sites and to dopamine melanin by three classes of binding sites. Both beef eye and dopamine melanin contained a small number of binding sites (n₁) with a high association constant (K₁) and a great number of binding sites (n₂ and n₃, respectively). The concordance between the association constants indicates a binding to identical sites on the two melanins. In addition, a small intermediary binding to dopamine melanin was found (n₂) which reflects certain differences in chemical structure between the two melanins -- beef eye melanin is obtained from tyrosine as precursor.

The total binding capacity (Σn) of beef eye melanin is 0.53 µmol/mg melanin. This is probably due to the higher content of carboxyl groups in beef eye melanin (Nicolaus, R.A., in Melanins, E. Lederer, Ed., Hermann, Paris (1968)). It is interesting to note that the total binding capacity of MPTP to beef eye melanin is of the same magnitude as that of chlorpromazine and cloroquine (Larsson et al., supra), two drugs that are known to give melanin related side effects.

Thus, it can be seen that melanin is an effective chelator of MPTP, a neurodegenerative disease-causing substance.

### EXAMPLE 2

### Protection from MPTP Induced Parkinson's Disease

The ability to protect a mammal from toxin-induced neurodegenerative disease, such as Parkinson's disease, is examined by treating monkeys with MPTP in a state in which MPTP could not bind to melanin.

Thirteen male monkeys (Macaca fascicularis) 5 to 8 years old, weighing 3.5 to 4.8 kg, are studied. Four animals are naive controls. Nine receive daily (0.35 mg per kg) injections of MPTP i.v. for four days. Three animals (M1-3) who received no chloroquine are the untreated controls. Six animals are pretreated with chioroquine (4 mg per kg) intramuscularly; three (S1-3) are pretreated for 12 days, and three (L1-3) for 24 days. All six pretreated animals continue to receive chloroquine injections during MPTP administration and for 10 days following MPTP exposure. The neurological examination evaluates the spontaneous movement, tremor, tone and deep tendon reflexes. A zero score in each category reflects a total loss of spontaneous movement, maximum tremor, maximum increase in tone, or maximum hyperreflexia. Deep tendon reflexes examined are brachial radials, knee jerk, and ankle jerk. Muscle tone tests evaluate protraction-retraction, abduction-adduction and flexion-extension in both upper and lower extremities. Tremor is rated on severity and the number of extremities involved. Spontaneous movement is evaluated in the morning over a 30 minute period. The rating scale consists of arbitrary units which are weighted to reflect the disturbances most prominent in MPTP-treated monkeys. Control values are the maximum (normal) score for each element of the exam and all control animals fell within 10% of the control values. The sum of scores for the four elements is multiplied by 5.26 to provide a total score with a value of 100 for control animals. The results are shown in Table 3.

**TABLE 3**

| Neurological Effects of Chloroquine on MPTP Neurotoxicity in Monkeys | | | | | |
|---|---|---|---|---|---|
| | Neurological Status | | | | |
| | Spontaneous Movement | Tremor | Tone | Reflexes | Total (x 5.26) |
| Control | 10 | 5 | 2 | 2 | 100 |
| MPTP | 1 | 1 | 0.5 | 0.5 | 16 |
| M1 | 1 | 1 | 2 | 1.5 | 29 |
| M2 | 1 | 0 | 0.5 | 0 | 8 |

| Short-Term Chloroquine/MPTP and MPTP | | | | | |
|---|---|---|---|---|---|
| S1 | 3 | 4 | 2 | 2 | 58 |
| S2 | 3 | 3 | 1 | 1 | 42 |
| S3 | 3 | 3 | 2 | 1 | 47 |

| Long-Term Chloroquine/MPTP and MPTP | | | | | |
|---|---|---|---|---|---|
| L1* | 0 | 2 | 1.5 | 1 | 24 |
| L2 | 5 | 5 | 1 | 1 | 63 |
| L3 | 7 | 5 | 2 | 2 | 84 |

| | | | | | |
|---|---|---|---|---|---|
| * Chloroquine level is 36% of that in other monkeys monitored. | | | | | |

The behavior of the monkeys receiving MPTP alone is similar to that of monkeys previously exposed to MPTP as described in other reports (Schwartzman, R. et al., Brain Res. 358, 137 (1985)). On the fifth day after MPTP exposure the animals manifest decreased mobility and spontaneous movement, abnormal posture, rigidity of the neck and limbs, increased muscle tone, hyperactive reflexes, tremor of upper extremities, and lack of vocalization (Table 3).

Five of the six monkeys pretreated with chloroquine are partially protected from MPTP-induced parkinsonian clinical symptoms. Of the three monkeys receiving long-term treatment with chloroquine, one animal (L-3) is almost completely protected except for a slight decrease in spontaneous movement. A second animal in this group (L-2) is also protected from the severe effects of MPTP. Although it exhibits modest rigidity, the monkey manifests no tremor, moves about the cage freely and vocalizes extensively. However, one animal (L-1) demonstrates motor deficits as severe as monkeys receiving MPTP alone; the reasons for the failure of chloroquine protection are described below. All three animals receiving short-term chloroquine pretreatment are partially protected from MPTP neurotoxicity; although they have some rigidity, all move about the cage readily, eat well, vocalize extensively and show only modest tremor.

To determine dopamine and homovanillic acid (HVA) levels, brain samples (10-20 mg wet weight) are homogenized in 300 µl of 0.4 M perchloric acid and centrifuged at 4°C for 10 minutes at 1,000 g. Aliquots of the supernatants are analyzed directly by reverse-phase high performance liquid chromatography (HPLC) on an ODS-3 column (Whatman Chemical Separation) with a Pellosil C₈ guard column (Alltech Associates). The mobile phase for this system is acetatephosphate/methanol (95:5) which includes EDTA and sodium heptanesulphonate as an ion-pairing agent (Bioanalytical Systems, Inc.). Detection is done electrochemically on a glassy carbon electrode (Bioanalytical Systems Inc.) at an applied voltage of 0.65 V. Plasma levels of chloroquine are determined by HPLC with UV detection. Samples are deproteinated with 0.2 vol. of 25% trichloroacetic acid followed by centrifugation at 4°C for 30 minutes at 1,000 g. Supernatants are removed, lyophilized overnight and reconstituted with 80 µl of 0.1 M perchloric acid. Samples are analyzed directly using reverse-phase HPLC with a Whatman ODS-3 column (Whatman) and Pellosil C₈ guard column (Alltech). The mobile phase for this system is 40% acetonitrile, 0.1 M sodium phosphate (pH 3.0) with 75 mM perchloric acid. Absorption is detected at 343 nm (Bergqvist, Y. et al., Chromat. 221, 2503 (1985)). Tyrosine hydroxylase (TH) activity is assayed by the tritium release method of Nagatsu, T. et al., Analyt. Biochem. 9, 122 (1964) and Levine, R. et al., Analyt. Biochem. 143, 205 (1984), employing modified reaction conditions of Coyle, J. Biochem. Pharmac. 21, 1935 (1972). Supernatant fluid (50 µl) from brain homogenate (1 g tissue in 20 vol 50 mM Tris, H 7.4) are added to 7 ml glass scintillation vials containing 5 µl 6 DL-6-methyl-5,6,7,8-tetrahydropterine (2.8 mg ml⁻¹), 5 µl FeSO₄ (2.78 mg ml ⁻¹) and 1 µC of ring labelled [³H]tyrosine. Mixtures are incubated 30 minutes at 37°C and the reaction terminated by adding 50 µl 3 M Na₂CO₃, pH 11.6. Toluene/isoamyl alcohol scintillant (5 ml) is then added directly to the vial and the contents mixed for 10 seconds. The results are shown in Table 4. The aqueous and organic phases are allowed to separate and the ³H₂O extracted into the organic phase determined.

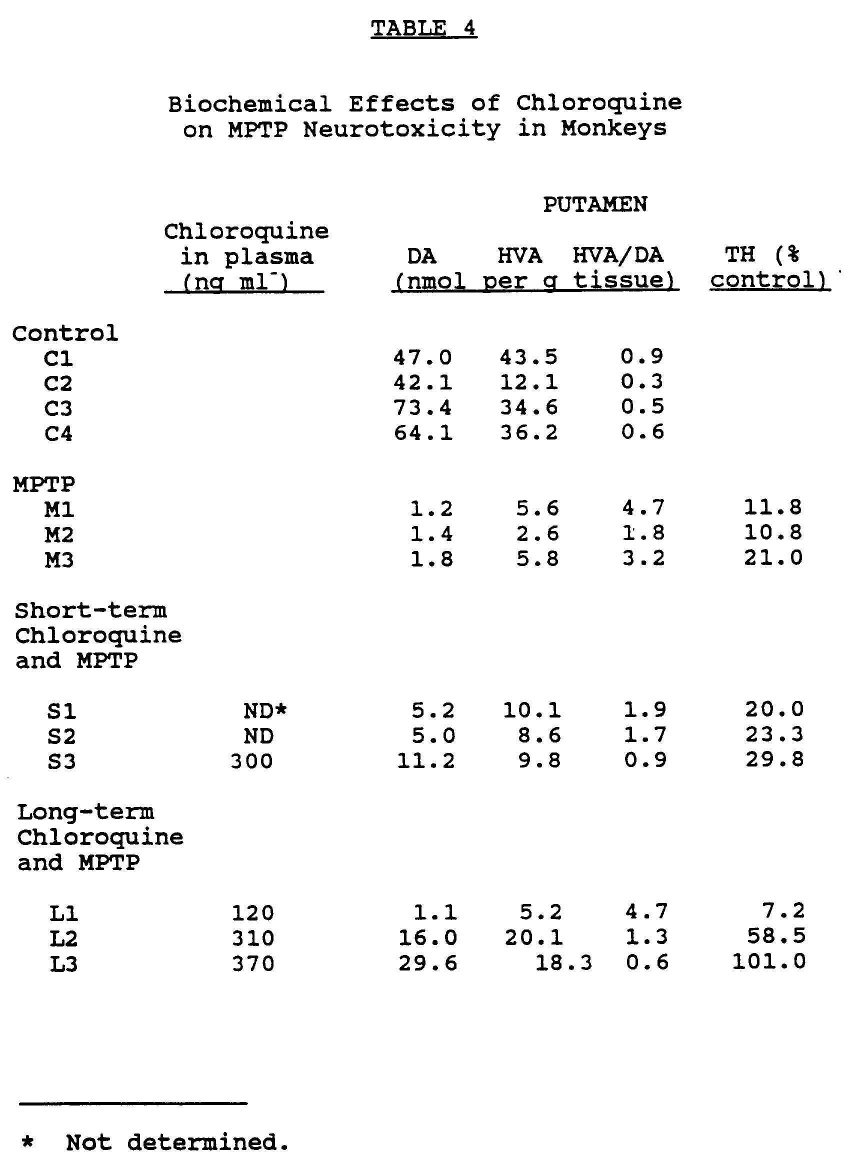

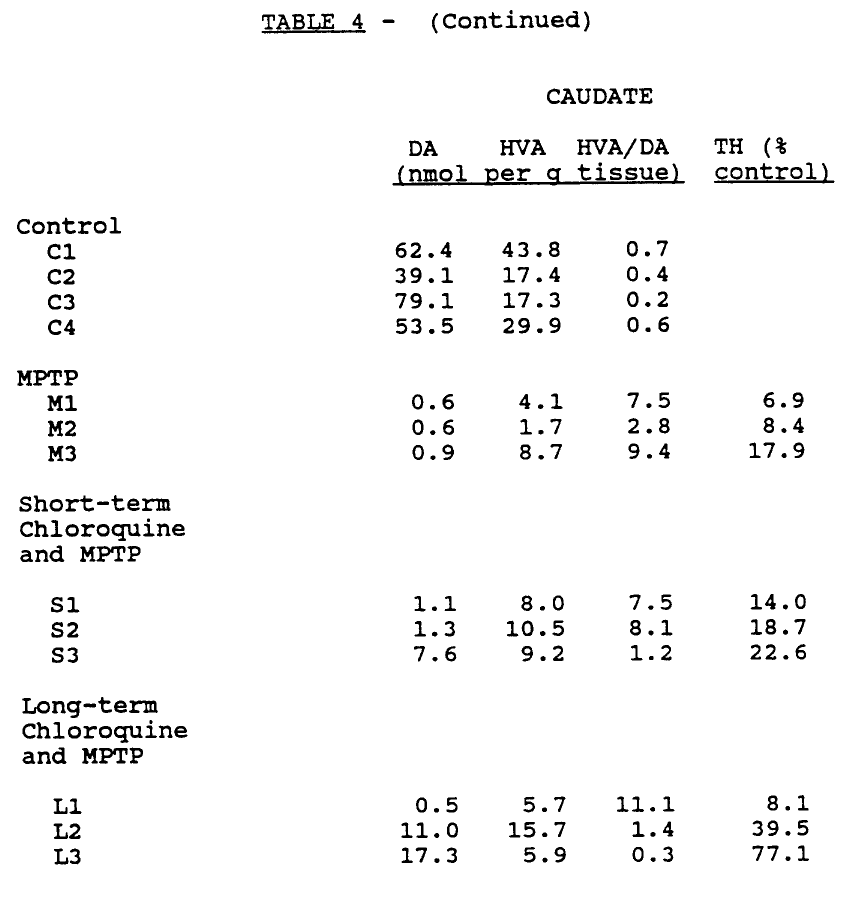

Results of neurochemical analyses closely parallel the clinical findings (Table 4). In monkeys receiving MPTP alone, amounts of dopamine, homovanillic acid (HVA) and tyrosine hydroxylase activity (TH) are markedly reduced in both the caudate and putamen. Dopamine is depleted to about 1% of control, whereas HVA was at about 10% of control. The resulting increased HVA/dopamine ratio in the MPTP animals presumably reflects the greater turnover of dopamine in residual dopamine neurons. In monkeys receiving MPTP alone, TH immunocytochemical preparations (Kitt, C.A. et al., Neuroscience 17, 1089 (1986)) reveals a pronounced reduction in the density of TH immunoreactive fibres and terminals in the putamen and to a lesser extent in the caudate nucleus as compared to controls (data not shown). The five chloroquine pretreated monkeys, which are clinically protected from MPTP neurotoxicity, show much slighter reductions in levels of dopamine and TH, as well as TH-immunoreactive fibres and terminals, than MPTP-treated animals.

Neuropathological findings fit well with the clinical and neurochemical observations. Representative neuromelanin-stained sections through the substantia nigra from each animal are rank-ordered for cell loss by two naive observers whose rankings are identical and closely parallel the neurochemical and clinical results. The correlation coefficient of the ranking with the caudate dopamine values (R) is 0.90. The greatest reduction in nigral cell number occurs in animals given MPTP alone (data not shown). Chloroquine-pretreated animals have more surviving neuromelanin-containing neurons, with the greatest number of cells remaining in the long-term pretreatment group.

Thus, short-term treatment with chloroquine provides partial protection against clinical, neurochemical and neuropathologic effects of MPTP, and in two of three animals, long-term treatment provides more pronounced protection. Why one of the monkeys receiving long-term chloroquine treatment (L-1) is not protected against the effects of MPTP is not known. Chloroquine in plasma is assayed in four of the monkeys immediately before administration of MPTP. Monkeys S-3, L-2 and L-3, which are protected against the effects of MPTP, have 300, 310 and 370 ng ml⁻¹ chloroquine respectively (≃1 µM of which half is bound to plasma protein). In contrast, monkey L-1, which developed a parkinsonian syndrome, has a plasma level of 120 ng ml⁻¹. Presumably, the failure of drug protection results from the diminished availability of chloroquine in this monkey.

The partial protection of monkeys from MPTP neurotoxicity elicited by chloroquine, together with the high-affinity interactions of MPP⁺ with neuromelanin (D'Amato, R.J. et al., Science 231, 987 (1986); D'Amato, R.J. et al., Neurochem. 48, 653 (1987)) indicates that destruction of dopamine neurons in the substantia nigra by exposure to low doses of MPTP is dependent upon interactions of MPP⁺ with neuromelanin. By inhibiting the binding of MPP⁺ to neuromelanin, chloroquine may reduce intraneuronal sequestration of MPP⁺, resulting in reduced toxicity to organelles such as mitochondria (Nickles, W.J. et al., Life Sci. 36, 2503 (1985)).

Example 1 demonstrates that melanin is capable of binding MPTP, a toxin which causes a neurodegenerative disease. Example 2 shows that the disease is caused by the binding of MPTP to melanin in the brain. Since melanin is capable of binding MPTP, it is evident that melanin which is administered to a mammal will bind an environmental neurotoxin such as MPTP, thus preventing a neurodegenerative disease such as Parkinson's disease.

### EXAMPLE 3

### Melanin Administration to Aid Neuron Recovery

Male C57BL/6J IMR mice 6-8 weeks of age are used throughout except in one experiment (see below) in which CB6F₁ [(BALB/cByJ IMR x C57BL/gJ IMR)F₁] mice of a similar age are used. Mice are housed five per cage in plexiglass cages with free access to food and water in a colony room maintained at 23 ± 1°C. Fluorescent lighting in the room is automatically turned on at 06.00 hours and off at 18.00 hours.

[³H]DA (31.6 Ci/mmol) and [³H]mazindol (19.6 Ci/mmol) are purchased from New England Nuclear (Boston, Massachusetts). MPTP is purchased from the Aldrich Chemical Company (Milwaukee, Wisconsin) and converted to the hydrochloride salt as described in Irwin, I. et al., Neurology 35, 619 (1985). Pargyline hydrochloride is a gift from Abbott Laboratories (Chicago, Illinois). Silver nitrate is purchased from Fisher Scientific Co. (Fairlawn, New Jersey). All other compounds are purchased from Sigma Chemical Co. (St. Louis, Missouri).

C57 black mice are administered MPTP hydrochloride intraperitoneally according to either one of two schedules: (1) 30 mg/kg/day for 10 days, or (2) 20 mg/kg/hour for 4 hours. The one group of CB6F₁ mice used in this study is administered MPTP according to the following schedule: 50 mg/kg/day for 13 days. This group is used only for anatomical studies looking for cell loss in the substantive nigra cells (SNc). All other studies are performed in C57 black mice.

MPTP hydrocloride is dissolved in distilled water at a concentration such that it could be injected at a desired dosage on a 1 ml/100 g body weight basis. Dose is expressed as the free base.

Melanin is isolated from Stretococcus antibioticus. Melanin is administered to the test mice at a dose of 10 mg/kg/day following the MPTP treatment by injection into the cerebrospinal fluid until the mice are killed.

The mouse striatum is obtained by placing the brain on its dorsal surface and making two coronal cuts; the first at the caudal end of the olfactory bulbs, the second at the level of the optic chiasma. After placing the resulting brain slice on its rostral surface, one horizontal cut is made just below the corpus callosum and another just above the anterior commissure. Remaining parietotemporal cortex is trimmed away using the external capsule as a landmark. Septal tissue lying between the caudate nuclei is removed by cutting along the tissue planes created by the frontal horns of the lateral ventricles. Striatal tissue thus isolated weighs approximately 20 mg per animal. Immediately after dissection, tissue is wrapped in aluminum foil and stored in liquid nitrogen until assay, with the exception of tissue for uptake studies which is used immediately.

The striatum is weighed, placed in a tube containing 1 ml of 0.4 normal perchloric acid, then homogenized with a Beckman polytron at a setting of 5 for 10 seconds. The homogenate is centrifuged at approximately 20,000 x g for 15 minutes. Concentrations of dopamine (DA), DOPAC and HVA in the supernatant are determined by reverse-phase liquid chromatography coupled with electrochemical detection according to the method of Mayer, G.S. et al., J. Chromatogr. 255, 533 (1983), with minor modifications. The mobile phase is prepared by mixing 965 ml of 0.15 M monochloroacetic acid with 35 ml of acetonitrile and adding 193 mg of sodium octyl sulfate. This solution is filtered and degassed and then 18 ml of tetrahydrofuran are added. Using this mobile phase at a flow rate of 1.3 ml/min, DA, DOPAC and HVA are resolved using a 4.6 mm x 25 cm C-18, 5 µ column (Brownless Labs). Detection and quantitation are performed using a dual series electrode detector (Coulochem Model 5100, Environmental Systems Associates, Wiggins, Mass.). Electrode potentials are set at +0.4 V (electrode 1) and -0.3 V (electrode 2). The response on electrode 2 is monitored (10 mV strip chart recorder) and used for quantitation relative to peak heights of known amounts of standards.

The in vitro accumulation of [³H]DA by crude striatal synaptosomal suspensions is measured using the method of Snyder, S.H. et al., J.Pharmacol.Exp.Ther. 165, 78 (1968) with minor modifications. Briefly, crude synaptosomal suspensions are prepared by homogenizing striatal tissue in 50 vols. (w/v) of ice-cold 0.32 M sucrose, then centrifuging the homogenate for 10 minutes at 1,000 x g. Aliquots (0.1 ml) of the supernatant are added over ice to tubes containing 1.9 ml of Krebs-Ringer phosphate buffer which contained these in final concentrations: 118 mM NaCl, 16.2 mM Na₂HPO₄, 4.7 mM KCl, 1.3 mM CaCl₂, 1.2 mM MgSO₄, 1.1 mM ascorbic acid, 11.1 mM glucose, 1.3 mM EDTA, <0.125 mM pargyline along with equimolar mixtures of [³H]DA and native DA at concentrations ranging from 0.025 to 0.5 µM. After vortexing, tubes (except temperature blanks) are incubated in a waterbath at 37°C for five minutes, then returned to ice. Synaptosomes are harvested by filtration. Filters are rinsed twice with 5 ml aliquots of physiological saline. Radioactivity in the filters is quantified by liquid scintillation spectroscopy. Assays are performed in sextuplicate at each DA concentration, with half of the samples serving as blanks. Active uptake is defined as the difference between [³H]DA (PM/mg tissue/5 min.) incubation at 37°C after correction for uptake at 0-4°C.

The binding of [³H]mazindol to striatal membranes is measured according to the method of Javitch, J.A. et al., Eur.J.Pharmacol. 90, 461 (1983).

Nerve terminal degeneration studies are performed using the method of Fink, R.P. et al., Brain Research 4, 369 (1967) (Procedure 1). This method makes possible selective silver impregnation of degenerating nerve fibers and terminals. Mice for these studies are killed under sodium pentobarbital anesthesia (40 mg/kg) by transcardial perfusion with 10% formal saline. The brain is immediately removed and stored in perfusion fluid at 0-4°C for at least one week before being sectioned on a freezing microtomee. 30 µm coronal sections are collected in 5% formal saline, then stained with silver according to Fink et al., supra. Mice for these studies are killed one and three days after treatment with either 20 mg/kg/h x 4 or 30 mg/kg/day x 10 of MPTP (n = 3 for each group) or 10 or 20 days after treatment with melanin.

Cell bodies in the SNc are examined in both frozen and paraffin embedded sections after fixation in 10% formal saline. Frozen sections (30 µm) are stained with silver according to Fink et al., supra. Mice for those studies are treated with either 20 mg/kg/h x 4 or 30 mg/kg/day x 10 of MPTP and killed one or three days after the last MPTP injection, at various intervals after treatment with melanin is initiated. Alternating serial paraffin sections (8 µm) through the entire SNc are stained with either hemotoxylineosin or luxol fast blue-cresyl violet. C57 black mice used in these studies are treated with 30 mg/kg/day x 10 of MPTP and killed 10 days after the last drug injection. CB6F₁ mice used in these studies are treated with 50 mg/kg/day x 13 and killed 21 days after the last drug injection.

The results which are obtained following MPTP treatment and melanin treatment after halting the MPTP treatment are discussed below.

Mice administered 30 mg/kg/day x 10 of MPTP and killed one week later show a 67% reduction in striatal DA content (Table 1). This result agrees well with that of Heikkila, R.E. et al., Nature 311, 467 (1984). Mice administered 20 mg/kg/h x 4 of MPTP show a comparable depletion of striatal DA (Table 5). The long-lasting depletion of DA induced by this shorter MPTP regimen is dose-related. No lethality is produced by the 2.5, 5 and 10 mg/kg/h x 4 MPTP regimens. Approximately 20% of the mice die after the 20 mg/kg/h x 4 regimen. Larger MPTP does regimens kill more than 50% of the animals. One day after cessation of MPTP treatment, mice administered with the 20 mg/kg four-hour MTP regimen or the 30 mg/kg 10 day regimen could not be distinguished behaviorally from their control littermates by casual observation.

**TABLE 5**

| Effect of 10-Day and 4-Hour MPTP Treatments on Mouse Striatal DA Content One Week Later | | | |
|---|---|---|---|
| Treatment | n | DA (µg/g) | % Depletion |
| Control | 10 | 10.7 ± 0.5 | -- |
| MPTP 30 mg/kg/day x 10 | 5 | 3.5 ± 0.3* | 67 |
| MPTP 20 mg/kg/h x 4 | 5 | 2.8 ± 0.5* | 74 |

| | | | |
|---|---|---|---|
| * Significantly different from control group (P <0.05; two-tailed Student's t-test). | | | |

Along with reduced level of DA, mice treated with 20/mg/kg/h x 4 of MPTP have decreased striatal concentrations of DOPAC and HVA. DOPAC is reduced from 0.96 (±0.14) µg/g to 0.28 (±0.02) µg/g and HVA from 1.38 (±0.05) µg/g to 0.60 (±0.06) µg/g (differences significant at 0.05 level). Mice administered 20 mg/kg/h x 4 of MPTP and killed one week later also show decreased striatal synaptosomal [³H]DA uptake (Table 6). The Vₘₐₓ was decreased by 62%. The Kₘ was not changed.

**TABLE 6**

| Kinetic Constants of [³H]DA Uptake One Week after MPTP | | |
|---|---|---|
| | Vₘₐₓ_{*} | Kₘ(µm) |
| Control | 5540 ± 480 | 0.14 ± 0.02 |
| MPTP | 2080 ± 305 ** | 0.12 ± 0.02 |

| | | |
|---|---|---|
| * Expressed as cpm[³H]DA/mg tissue/5 min. | | |
| ** Significantly different from control. | | |

The [³H]mazindol binding site has recently been proposed as an additional dopaminergic terminal marker. Mice administered 20/mg/h x 4 of MPTP and killed three weeks later also show a decreased number of [³H]mazindol binding sites (Table 7). The Bₘₐₓ was reduced by 44%. The K₄ was unchanged.

**TABLE 7**

| Kinetic Constants of [³H]Mazindol Binding to Striatal Membranes Three Weeks After MPTP | | |
|---|---|---|
| | Bₘₐₓ_{*} | K₄(nm) |
| Control | 361 | 17.6 |
| MPTP 20 mg/kg/h x 4 | 201 | 17.3 |

| | | |
|---|---|---|
| * Expressed as pmol/g tissue. | | |

Three of three mice administered 20 mg/kg/h x 4 of MPTP and killed one day later for silver degeneration studies show a large amount of fine granular argyrophilic debris in their striata. Some fine granular degeneration is also found in the nucleus accumbens and olfactory tubercle, but in these regions it was much less dense. No such degeneration is found in identically treated sections of control mice, or in other brain regions visible in coronal brain sections at the level of the striatum. None of three mice treated with 20 mg/kg/h x 4 of MPTP but pretreated with 25 mg/kg of pargyline, which blocks the dopaminergic neurochemical deficits induced by MPTP in mice (Heikkila, R.E. et al., supra) show any evidence of striatal terminal degeneration.

In frozen sections through the SNc stained with silver according to the Fink-Heimer method, two of the same three mice which show dense terminal degeneration in their striata show no sign of cell body destruction. The third animal has a few SNc cells which may have been undergoing degeneration. These few cell bodies stain intensely with silver, appear shrunken, and some have dendritic arbors which were argyrophilic and appear beaded. Cells with similar appearance have been interpreted as undergoing degeneration by various authors. Although formal counts of these neurons were not performed, affected neurons appear to represent only a very minor fraction of the total SNc cell population. In serial paraffin sections through the entire length of the SNc, there is no definite cell loss or glial reaction in C57 black mice treated with 30 mg/kg/day x 10 (n = four experimental, two controls) or in CB6F₁ mice treated with 50 mg/kg/day x 13 (n = four experimental and four controls) of MPTP. Coded sections from control and experimental animals cannot be distinguished from each other by either of two observers. Mice from these two groups were killed 10 and 21 days, respectively, after drug treatment so as to optimize the possibility of detecting cell loss.

Determination of the level of striatal DA, its metabolites, and synaptosomal uptake at various times after 20 mg/kg/h x 4 of MPTP reveals that substantial recovery in all of these parameters occurs with time. DA level rises from 28% of control one week after MPTP to 69% of control 15 months later. Three months after MPTP, there is still a 34% depletion of striated DA. Partial recovery of striatal DA also occurs after a 30 mg/kg/day x 10 MPTP regimen. [³H]DA uptake capacity likewise recovered with time. The Vₘₐₓ of [³H]DA striatal uptake increases from 37% of control one week after MPTP to 79% of control three months later (6238 (± 520) CPM [³H]DA/mg tissue/5 min in control mice vs. 4928 (± 408) CPM [³H]DA/mg tissue/5 min in MPTP mice). Over this same time period, DOPAC rises from 29% of control three months later (1.53 ±0.09 µg/g in controls vs. 1.03 ± 0.03 µg\g in MPTP mice). HVA rises from 43% of control one week after MPTP (vida supra) to 80% of control three months later (1.36 ± 0.11 µg/g in control vs. 1.09 ± 0.04 µg/g in MPTP mice).

When melanin is given following MPTP treatment, the time period required for a similar recovery is reduced and recovery continues through the five-month examination. For example, the Vₘₐₓ of [³H]DA striatal uptake increases to 75% of control after 3.5 months of melanin treatment and increases to 85% after five months of melanin treatment.

These results clearly show that during the period tested, melanin is capable of aiding the recovery of neurons following an injury to the neurons. Since melanin is capable of aiding the recovery of neurons following an injury, melanin can be used to treat Alzheimer's disease.

### EXAMPLE 4

### Melanin Treatment of Parkinson's Disease

Male squirrel monkeys (aged 2-3 years) are used for this study. MPTP (Delmar Chemicals) is converted to its hydrochloride salt, dissolved in sterile water to a final concentration of 1 mg/ml (as the free base) and filtered through a 0.22 µm millipore filter into sterile injectable vials. All injections are intraperitoneal.

Three different dosage schedules of MPTP are used. Monkey Group A receives four doses, 2 mg/kg each, which are given at two-hour intervals. Monkey Group B is treated over a five-day period. On day 1, a single 2 mg/kg dose is given. On day 3, two injections of 2 mg/kg each are given, six hours apart. On day 5, a 3 mg/kg dose is given followed by a 0.5 mg/kg dose four hours later (total dose: 9.5 mg/kg). Three doses of 3 mg/kg each are given to Monkey Group C, spaced at six-day intervals. Monkey Group D serves as a control.

After two or more doses of MPTP, increasing bradykinesia and frequent "nodding off" (characterized by closing of the eyes and a slow downward drift of the head) are observed in all animals. Fasciculations of the thigh muscles occur in Monkey Group A. A transient but striking behavioral syndrome is seen after each of the last three doses in Monkey Group A, and after the final two doses in Monkey Group B. This syndrome is characterized by repeated abrupt eye opening and shaking and extension of the extremities.

All monkeys eventually become profoundly akinetic, usually sitting hunched over in a tightly flexed posture. They exhibit a generalized increase in tone. Vocalization and oral intake were markedly diminished. Monkeys hold awkward postures for lengthy periods, and sometimes freeze in the middle of a movement. They are often unable to release their grip, getting stuck on the bars of the cage. Tremor and a flexed posture of the arms are seen in Monkey Group C.

Two days after receiving MPTP, one monkey in Group A is given one-fourth of a 2.5 mg bromocriptine tablet (Parlodel®) and one-eighth of a 10/100 carbidopa/L-dopa combination tablet (Sinemet®) orally. Within 30 to 60 minutes, the animal is fully mobile and appears almost normal for five hours. A similar response to the same treatment is observed on each of the next four days. Subsequently, the animal becomes less responsive to medication and is sacrificed on day 10. One monkey in Group B responds to Sinemet® (one-eighth of a 10/100 tablet) with full mobility on day 9. However, on subsequent days, he becomes increasingly less responsive to medication, often appearing uncoordinated and shaky. This monkey is sacrificed on day 15. One monkey in Group C becomes nearly normal for 24 hours after a single dose of Sinemet® (10/100) on day 25. Three days later, this monkey becomes profoundly hypokinetic, develops slow respirations, and dies.

After increased bradykinesis and frequent nodding-off are observed in the monkeys, several monkeys of Groups A, B and C are administered melanin by injection into the cerebrospinal fluid at a dose of 50 mg/kg daily. The melanin is isolated from Streptococcus antibioticus. Amelioration of the bradykinesia and rigidity are seen in the melanin-treated animals. The monkeys' overall functional ability and secondary motor manifestations also improved during the course of the melanin treatment.

### EXAMPLE 5

### Preparation of Cloned Human Tyrosinase

Cloned human tyrosinase is prepared using the method of Kwon, B.S. as described in the published PCT application WO 88/02372.

The tyrosinase is produced in E. coli strain MM 294. The λmel 34 cDNA (as described by Kwon, B.S. in the same PCT application) is fused to a Tac expression vector (U.S. Pharmacia Inc.) which has Trp and lac promotor together. The construct is expressed in the E. coli strain MM 294 and subsequently purified by affinity column chromatography.

This tyrosinase is then used to manufacture a medicament to treat neurodegenerative diseases of the nervous system.

### EXAMPLE 6

### Introduction of Human Tyrosinase Gene Into a Defective HSV-1 Vector

A defective herpes simplex virus 1 (HSV-1) vector, pHSVlac, has been developed by Geller, A.I., et al., Science 241, 1667 (1988). This vector is useful for transporting genes through the blood brain barrier.

The vector, pHSVlac, contains the Escherichia coli lacZ gene which is under the control of the HSV-1 immediate early 4/5 promoter. Using conventional publicly available endonucleases, pHSVlac is digested at its EcoRI sites to remove the E. coli lacZ gene. The λ mel 34 human tyrosinase gene (described by Kwon, B.S. in PCT application WO 88/02372) is then inserted to pHSVlac in place of the E. coli lacZ gene, and the vector is religated using conventional techniques.

This chimeric pHSVlac vector may then be used to introduce the tyrosinase gene into patients suffering from diseases caused by a melanin deficiency.

### EXAMPLE 7

### Stably Transforming Cultured Peripheral Neurons with the pHSVlac Vector Expressing Tyrosinase Gene

Primary cultures of dissociated neurons from dorsal root ganglia and superior cervical ganglia of newborn rats are prepared in accordance with the techniques taught by Hawrot, E. et al., Methods Enzymol. 58, 574 (1979). The cultures are then infected with the chimeric pHSVlac vector of Example 6, above, and incubated for 24 hours at 37°C. The cultures are then fixed and assayed for tyrosinase using antityrosinase antibodies (available from Dr. Seymour H. Pomerantz, Department of Biological Chemistry, University of Maryland School of Medicine, Baltimore, Maryland 21201) and conventional techniques. Tyrosinase is found to be present in both the dorsal root ganglia cell cultures and the superior cervical ganglia cell cultures.

### EXAMPLE 8

### Transneuronal Transfer of the pHSVlac Vector Expressing Tyrosinase Gene

In accordance with the technique of Ugolini et al., Science 243, 89 (1989), eight rats (6 to 7 weeks old) are unilaterally injected in the ulnar and median nerves with the chimeric pHSVlac vector of Example 6, above. After four days, the rats are anesthetized and perfused with 10% Formalin as taught by Ugolini et al., Brain Res. 442, 242 (1987). The brains and spinal cords of the rats are cut into 60 µm transverse frozen sections, and the presence of tyrosinase is assayed using antityrosinase antibodies and conventional techniques as described in Example 7, above. Tyrosinase is found to be present in the rat brain neurons, due to the transneuronal transfer of the chimeric pHSVlac vector from its peripheral neuron injection site to the brain.

## Claims

1. Use of an active substance, which causes an increase in the concentration of melanin in the tissue, said active substance selected from the group consisting of melanin; melanin derivatives in combination with melanin, tyrosinase, tyrosinase gene or melanin-concentrating hormone; tyrosinase; tyrosinase gene; melanin-concentrating hormone and combinations thereof, in the manufacture of a medicament to treat degenerative diseases of the nervous system.

2. The use of claim 1, wherein the neurological dysfunction or disorder is caused upon exposure to neurodegenerative disease-causing substances.

3. The use of claim 1, wherein said disease is selected from the group consisting of senile dementia, Alzheimer's disease and Pick's disease.

4. The use of claim 1, wherein said disease is selected from the group consisting of Huntington's chorea, cerebrocerebellar degeneration, amaurotic family idiocy (neuronal lipidoses), leukodystrophy, familial myoclonus epilepsy, and Wilson's disease (hepatolenticular degeneration, Westphal-Strumpell pseudosclerosis).

5. The use of claim 1, wherein said disease is selected from the group consisting of paralysis agitans (Parkinson's disease), dystonia musculorum deformans (torsion dystonia), Hallervorden-Spatz disease and other restricted dyskinesias, familial tremor and spasmodic torticollis.

6. The use of claim 1, wherein said disease is selected from the group consisting of cerebellar degenerations and spinocerebellar degenerations (Friedreich's ataxia, Marie's hereditary ataxia).

7. The use of claim 1, wherein said disease is selected from the group consisting of amyotrophic lateral sclerosis, progressive muscular atrophy, progressive bulbar palsy, primary lateral sclerosis, infantile muscular atrophy (Werdnig-Hoffmann disease), other forms of familial progressive muscular atrophy (including Wohlfart-Kugelberg-Welander syndrome), hereditary spastic paraplegia, progressive neural muscular atrophy, peroneal muscular atrophy (Charcot-Marie-Tooth), hypertrophic interstitial neuropathy (Dejerine-Sottas), and miscellaneous forms of chronic progressive neuropathy.

8. The use of claim 1, wherein said disease is selected from the group consisting of hereditary optic atrophy (Leber's disease) and pigmentary degeneration of the retina (retinitis pigmentosa).

9. The use of claim 1, wherein said disease is selected from the group consisting of depression and schizophrenia.

10. The use of claim 2, wherein said active substance is administered before or immediately after said exposure.

11. The use of claim 1, wherein said active substance is used in the manufacture of a medicament to assist the repair of neurons in a mammal having neuron damage.

## Patentansprüche

1. Verwendung einer aktiven Substanz, welche einen Anstieg in der Melanin-konzentration im Gewebe verursacht, wobei die aktive Substanz ausgewählt ist aus der Gruppe bestehend aus Melanin; Melaninderivaten in Kombination mit Melanin, Tyrosinase, Tyrosinasegen oder Melanin-konzentrierendem Hormon; Tyrosinase; Tyrosinasegen; Melanin-konzentrierendem Hormon und Kombinationen davon zur Herstellung eines Medikaments, um degenerative Erkrankungen des Nervensystems zu behandeln.

2. Verwendung nach Anspruch 1, wobei die neurologische Funktionsstörung oder Erkrankung durch Einwirken von Substanzen verursacht wird, die neurodegenerative Erkrankungen verursachen.

3. Verwendung nach Anspruch 1, wobei die Erkrankung ausgewählt ist aus der Gruppe bestehend aus seniler Demenz, Alzheimer-Krankheit und Pick-Krankheit.

4. Verwendung nach Anspruch 1, wobei die Erkrankung ausgewählt ist aus der Gruppe bestehend aus Huntington-Chorea, zerebrozerebellärer Degeneration, amaurotischer familiärer Idiotie (neuronale Lipidosen), Leukodystrophie, familiärer Myoklonusepilepsie und Wilson-Krankheit (hepatolentikuläre Degeneration, Westphal-Strumpell-Pseudosklerose).

5. Verwendung nach Anspruch 1, wobei die Erkrankung ausgewählt ist aus der Gruppe bestehend aus Schüttellähmung (Parkinson-Krankheit), Dystonia Musculorum Deformans (Torsionsdystonie), Hallervorden-Spatz-Krankheit und anderen beschränkten Dyskinesien, familiärem Tremor und spastischem Schiefhals (Torticollis spasticus).

6. Verwendung nach Anspruch 1, wobei die Erkrankung ausgewählt ist aus der Gruppe bestehend aus zerebellären Degenerationen und spinozerebellären Degenerationen (Friedreich-Ataxie, Marie-Syndrom).

7. Verwendung nach Anspruch 1, wobei die Erkrankung ausgewählt ist aus der Gruppe bestehend aus amyotrophisch lateraler Sklerose, progressiver muskulärer Atrophie, progressiver bulbärer Lähmung, primärer lateraler Sklerose, infantiler muskulärer Atrophie (Werdnig-Hoffmann-Krankheit), anderen Formen familiärer progressiver muskulärer Atrophie (einschließlich Wohlfahrt-Kugelberg-Welander-Syndrom), erblicher spastischer Paraplegie, progressiver neuraler Muskelatrophie, peronäaler Muskelatrophie (Charcot-Marie-Tooth), hypertropher interstitieller Neuropathie (Dejerine-Sottas), und gemischten Formen chronischer progressiver Neuropathie.

8. Verwendung nach Anspruch 1, wobei die Erkrankung ausgewählt ist aus der Gruppe bestehend aus erblicher optischer Atrophie (Leber-Krankheit) und pigmentärer Degeneration der Retina (Retinitis pigmentosa).

9. Verwendung nach Anspruch 1, wobei die Erkrankung ausgewählt ist aus der Gruppe bestehend aus Depression und Schizophrenie.

10. Verwendung nach Anspruch 2, wobei die aktive Substanz vor oder unmittelbar nach der Einwirkung verabreicht wird.

11. Verwendung nach Anspruch 1, wobei die aktive Substanz bei der Herstellung eines Medikaments verwendet wird, das die Reparatur von Neuronen in Säugern mit Neuronschäden unterstützt.

## Revendications

1. Utilisation d'une substance active, qui provaque une augmentation de la concentration de mélanine dans le tissu, ladite substance active étant choisie dans le groupe constitué de la mélanine ; des dérivés de la mélanine en combinaison avec de la mélanine, de la tyrosinase, du signe de tyrosinase ou de l'hormone de concentration de mélanine; de la tyrosinase ; de gène de tyrosinase ; d'hormone de concentration de mélanine et de combinaisons de ceux-ci, dans la fabrication d'un médicament pour traiter des maladies dégénérescentes du système nerveux.

2. Utilisation selon la revendication 1, dans laquelle la dysfonction au le dérèglement est provoqué au cours d'une exposition à des substances provoquant des maladies neuro-dgénératrices.

3. Utilisation selon la revendication 1, dans laquelle ladite maladie est choisie dans le groupe composé de la démence sénile, de la maladie d'Alzheimer et de la maladie de Pick.

4. Utilisation selon la revendication 1, dans laquelle ladite maladie est choisie dans le groupe composé de la chorée de Huntington, de la dégénérescence cérébrocérébelleuse, de l'idiotie amaurotique familiale (lipidoses neuroniques), de la leucodystrophie, de la myocolie épileptique progressive familiale, et de la maladie de Wilson (dégénérescence hépatolenticulaire, pseudosclérose de Westphal-Strumpell).

5. Utilisation selon la revendication 1, dans laquelle ladite maladie est choisie dans le groupe composé de la paralysie agitante (maladie de Parkinson), de la dystonie musculaire déformante (torsion dystonia, maladie de Ziehen-Oppenheim), de la maladie de Hallevorden-Spatz et d'autres dyskinésies restreintes, du tremblement familial et du torticolis spasmodique.

6. Utilisation selon la revendication 1, dans laquelle ladite maladie est choisie dans le groupe composé des dégénérescences cérébelleuses et des degénérescences spino-cérébelleuses (ataxie de Friedreich, ataxie héréditaire de Charcot-Marie).

7. Utilisation selon la revendication 1 dans laquelle ladite maladie est choisie dans le groupe composé du tabès dorsal spasmodique amyotrophique, de l'amyotrophie musculaire progressive, de la paralysie bulbaire progressive, de la paralysie labio-glosso-pharyngée, du tabès dorsal spasmodique primitif, de la poliomyélite antérieure chronique familiale de l'enfant (amyotrophie à forme de Werdnig-Hoffmann), et d'autres de formes d'atrophie musculaire progressive de l'enfance (comprenant le syndrome Kugelberg-Welander), la paraplégie spasmodique héréditaire, l'amyotrophie primitive progressive, l'atrophie péronière (maladie de Charcot-Marie-Tooth), la névrite hypertrophique familiale (maladie du type Déjerine-Sottas) et les diverses formes de neuropathie progressive chronique.

8. Utilisation selon la revendication 1, dans laquelle ladite maladie est choisie dans le groupe composé de l'amaurose tapéto-rétinienne (maladie de Leber) et de la dégénérescence de pigmentation de la rétine (rétinite pigmentaire, retinitis pigmentosa).

9. Utilisation selon la revendication 1, dans laquelle ladite maladie est choisie dans le groupe composé de la dépression et de la schizophrénie.

10. Utilisation selon la revendication 2, dans laquelle ladite substance active est administrée avant ou immédiatement après ladite exposition.

11. Utilisation selon la revendication 1, dans laquelle ladite substance active est utilisée dans la fabrication d'un médicament pour aider à la réparation de neurones chez un mammifère ayant une lésion des neurones.
